Europäisches Patentamt

European Patent Office

Office européen des brevets

(19)

(11) Veröffentlichungsnummer: **0 238 993 B1**

(12) **EUROPÄISCHE PATENTSCHRIFT**

(45) Veröffentlichungstag der Patentschrift: **31.05.95**

(21) Anmeldenummer: **87103942.6**

(22) Anmeldetag: **18.03.87**

(51) Int. Cl.⁶: **C12N 15/00**, C12P 21/02, C07K 14/00, C12N 1/20, A61K 38/55

(54) **Gentechnologisch-hergestellte Aprotinin-Homologe.**

(30) Priorität: **26.03.86 GB 8607523**

(43) Veröffentlichungstag der Anmeldung: **30.09.87 Patentblatt 87/40**

(45) Bekanntmachung des Hinweises auf die Patenterteilung: **31.05.95 Patentblatt 95/22**

(84) Benannte Vertragsstaaten: **AT BE CH DE ES FR GB IT LI NL SE**

(56) Entgegenhaltungen:
EP-A- 244 627      EP-A- 0 132 732
EP-A- 0 157 235      EP-A- 0 171 024
EP-A- 0 207 402      WO-A-86/01229

**JOURNAL OF CELLULAR BIOCHEMISTRY, Suppl. 9B, 1985, Abstracts of 14th Annual Meetings, 9. März- 4. April 1985, Seite 95, Ref. Nr. 0641 A.R. Liss, Inc., New York, US, C.B. MARKS et al.**

**CHEMICAL ABSTRACTS, Band 84, 1976, Seite 229, Ref. Nr. 101435K, Columbus, Ohio, US, H. JERING et al.**

(73) Patentinhaber: **BAYER AG**

**D-51368 Leverkusen (DE)**

(72) Erfinder: **Auerswald, Ernst-August, Dr.**
**Michael Beer Str. 10**
**D-8000 München 60 (DE)**
Erfinder: **Schröder, Werner, Dr.**
**Claudiusweg 9**
**D-5600 Wuppertal 1 (DE)**
Erfinder: **Schnabel, Eugen, Dr.**
**Schimmelweg 6**
**D-5600 Wuppertal 11 (DE)**
Erfinder: **Bruns, Wolfgang, Dr.**
**Kaiser-Wilhelm-Allee 37**
**D-5600 Wuppertal 1 (DE)**
Erfinder: **Reinhardt, Gerd, Dr.**
**Am Eckbusch 55a**
**D-5600 Wuppertal 1 (DE)**
Erfinder: **Kotick, Michael, Dr.**
**54231 Old Mill Drive**
**Elkhart IN 46514 (US)**

EP 0 238 993 B1

PROC. NATL. ACAD: SCI. USA, Band 80, Nr. 22, November 1983, Seiten 6838-6842, Washington US S. ANDERSON et al.

THE EMBO JOURNAL, Band 5, Nr. 12, 1986, Seiten 3219-3225, IRL Press Ltd, Oxford, GB, B. von WILCKEN-BERGMAN et al.

## Beschreibung

Die Erfindung betrifft Aprotinin-Homologe und deren Herstellung mit Hilfe der rekombinanten DNA-Technologie.

Die chemisch synthetisierten DNA-Moleküle, wie sie hier offenbart sind, werden durch die DNA-Sequenz charakterisiert, die für neue Polypeptide oder für Polypeptide, die im wesentlichen in der Aminosäuresequenz und Zusammensetzung von Aprotinin oder Aprotinin-Homologen übereinstimmen, und die die biologische Aktivität von Aprotinin oder von Aprotinin-Homologen haben, kodieren.

Aprotinin ist ein bekanntes Peptid mit 58 Aminosäuren und besitzt eine inhibierende Wirkung auf Trypsin, Chymotrypsin, Plasmin und Kallikrein. Es ist ein basischer Proteinaseinhibitor aus Organen des Rinds und wurde zu einem wertvollen Arzneimittel, genannt Trasylol®, für die Behandlung verschiedener Erkrankungen, z. B. hyperfibrinolytische Blutung und traumatisch-hämorragischer Schock (zur Übersicht siehe H. Fritz und G. Wunderer, 1983, Drug. Res. 33, 479 - 494).

Es wurde kürzlich gezeigt, daß Homologe von Aprotinin mit anderen Aminosäuren in der Position 15, anstelle von Lysin, wertvolle Proteinaseinhibitoren darstellen, die im Vergleich zu Aprotinin eine modifizierte Wirkung und Wirksamkeit besitzen (DE-OS 33 39 693 entspricht EP 132 732; H. R. Wenzel et al.)
1985 in Chemistry of Peptides and Proteins, Band 3). Diese Aprotinin-Homologen besitzen eine stark inhibierende Wirkung auf Elastasen aus Pankreas und Leukocyten, sowie auf Cathepsin G.

Diese Homologen von Aprotinin können therapeutisch bei Erkrankungen im Zusammenhang mit übermäßiger Freisetzung pankreatischer Elastase (Pankreatitis), Serumelastase (Arteriosklerose), Leukozytenelastase bei akuten und chronischen Entzündungen mit einer Schädigung des Bindegewebes, bei Schäden der Gefäßwände, bei nekrotischen Erkrankungen und bei einer Degenerierung des Lungengewebes verwendet werden. Ebenso wichtig ist ihre Rolle, im Zusammenhang mit lysosomalen Enzymen, insbesondere Leukozytenelastase, und zwar bei Entzündungsreaktionen aufgrund immunologischer Prozesse, z.B. bei rheumatoider Arthritis.

Obwohl Aprotinin und Aprotinin-Homologe aus Organen des Rinds und durch halbsynthetische Umwandlung von Rindertrypsininhibitor (Tschesche, M., Wenzel, M.,Schmuck, R., Schnabel, E. DE-OS 33 39 693 A1 vom 15.5.85) erhalten werden können, sind die Ausbeuten doch recht gering.

Es wurde erkannt, daß die Anwendung von rekombinanter DNA-Technologie sowie assoziierter Technologien einen wirksamen Weg darstellt, um die erforderlichen großen Mengen an Aprotinin-Homologen hoher Qualität zur Verfügung zu stellen. Das Ziel war, Aprotinin-Homologe, die biologisch aktiv sind, als Produkte der rekombinanten DNA-Technologie aus einem Wirtsorganismus zu erhalten.

Methoden zur Expression heterologer DNA in einem Mikroorganismus sind bekannt.

DNA, die für Polypeptide mit bekannten Aminosäuresequenzen kodiert, kann entweder unter Verwendung der genomischen DNA-Sequenz oder der c-DNA-Sequenz, die komplementär zur mRNA ist, oder durch die Wahl von Codons gemäß dem genetischen Code und Herstellung eines synthetischen Gens, gewonnen werden.

Eine partielle DNA-Sequenz aus dem Rinder-Genom, die das Rinder-Pankreastrypsininhibitor-Gen enthält, wurde von S. Anderson und I.B. Kingston, 1983, Proc. Natl. Acad. Sci. USA, 80, 6838 - 6842 kloniert, um einen genomischen Klon für BPTI zu charakterisieren.

Ein großer Abschnitt des Rindergenoms, der für BPTI und Rinder-Milzinhibitor II kodiert, wurde kürzlich sequenziert (Kingston, I.B. und Anderson, 1986, Biochem. J. 233, 443-450).

Aufgabe der vorliegenden Erfindung ist es, Aprotinin-Homologe, Nukleinsäuren, die dafür kodieren, Vektoren, welche diese Nukleinsäuren enthalten und damit transformierte Zellen, sowie Verfahren zum Erhalt von Aprotinin-Homologen zur Verfügung zu stellen. Im vorliegenden Falle war es besonders vorteilhaft, für die Herstellung synthetischer Gene so optimal auszuwählen, daß sie vielseitig angewendet werden können.

Dies ist insbesondere bei der Konstruktion eines synthetischen Master-Gens der Fall, welches DNA-Blöcke oder Kassetten umfaßt, die von einer nur einmal vorkommenden Erkennungsstelle von Restriktionsenzymen begrenzt werden, Ein solches Genkonstrukt erlaubt die einfache Modifizierung oder Mutation aller DNA-Sequenzen innerhalb solcher DNA-Blöcke.

Aprotinin-Homologe wurden durch rekombinante DNA-Technologie hergestellt. Diese Aprotinin-Homologen, wie z.B. Val-15-, Ile-15-, Leu-15-, Phe-15- und Ala-15-, Arg-15-, Gly-15-, Ser-15-, Trp-15-, Tyr-15-, Aprotinin in Kombination mit einer Substitution in Position 52 durch Glu, Leu, Val, Ser oder Thr werden als äquivalent angesehen mit bekanntem Aprotinin oder dessen Homologen, die in Position 52 Met haben und zusammen mit ihrer Herstellung offenbart sind. Die Substitution von Met-52 erlaubt die Herstellung von Aprotinin oder Aprotinin-Homologen, in welchen ein durch Gentechnologie fusioniertes Polypeptid durch Bromcyan am Met in den fusionierten Polypeptiden gespalten wird.

Die synthetische DNA, die für solche Homologe kodiert, rekombinante Plasmide, die Strukturgene zur Expression von Homologen umfassen, und E. coli, die mit rekombinanten Plasmiden transformiert sind, werden ebenfalls offenbart.

Im folgenden wird die Strategie zur Konstruktion und zur Selektion von DNA-Fragmenten, die für Aprotinin und Aprotinin-Homologe kodieren, gezeigt.

Die bekannte Proteinsequenz und der genetische Code von Aprotinin und Aprotinin-Homologen wurden verwendet, um eine DNA-Sequenz, die für solche Polypeptide kodiert, zu bestimmen.

Die Degenerierung des genetischen Codes gewährt eine bestimmte Freiheit in der Wahl von Codons für eine jede gegebene Aminosäuresequenz.

Alle möglichen Basensubstitutionen unter den Codons, die die Aminosäuresequenz dieses Proteins kennzeichnen, wurden bestimmt. Dementsprechend wurden alle potentiellen Restriktionsstellen, die innerhalb der möglichen DNA-Sequenzen lokalisiert sind, bestimmt.

Die Codonwahl für Master-Gene wurde von folgenden Überlegungen begleitet:

1. Codons und Fragmente wurden ausgewählt, und die Zusammensetzung von Fragmenten wurde so geplant, daß eine unnötige Komplementarität der Fragmente vermieden wird.

2. Regionen mit häufiger A-T-Basenpaarung werden vermieden, um die Schwierigkeiten durch eine vorzeitige Terminierung der Transkription zu überwinden.

3. Restriktionsstellen wurden so gewählt, wie es zur Erleichterung der Verifikation von Transformanten oder Basensubstitutionen durch Austausch geeigneter Fragmente mit anderen Fragmenten erforderlich war, so daß man leicht Modifikationen von Aprotinin herstellen und die Beziehung zwischen solchen Strukturen und deren qualitativen und quantitativen Wirkungen bestimmen kann.

4. Die Mehrheit der gewählten Codons sind die, wie sie für die Expression mikrobieller Genome bevorzugt sind (s. H. Grosjean udn W. Fiers, Gene, 18 (1982) 192-209; M. Gouy und C. Gautier, Nucleic Acids Research, 10 (1982) 7055 - 7074).

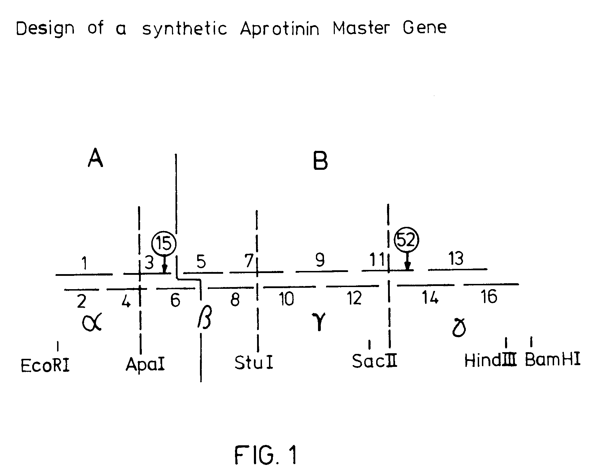

Das prinzipielle Konstrukt für synthetische Aprotinin-Gene und deren Homologe ist in Fig. 1 wiedergegeben.

Das Konstrukt des synthetischen Master-Gens, das aus vier Blöcken besteht (genannt $\alpha$, $\beta$, $\gamma$, $\delta$), umgeben von Erkennungsstellen für Restriktionsendonukleasen, erlaubt eine leichte Modifizierung und Änderung der DNA-Sequenzen (Codongebrauch, Mutationen, Proteinengineering, Verstärkung von Genen) für nicht-fusionierte und fusionierte Expressionen.

Die synthetischen Gene würden wie folgt konstruiert:

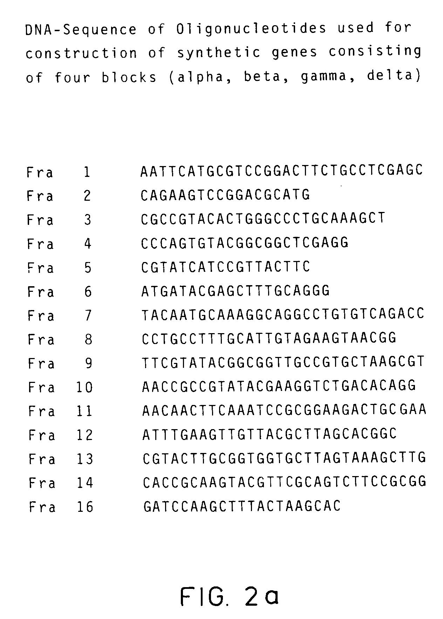

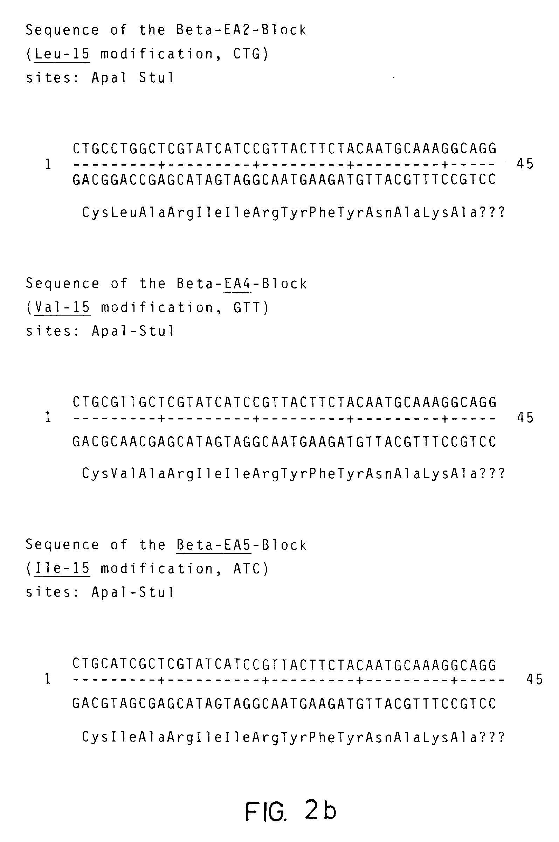

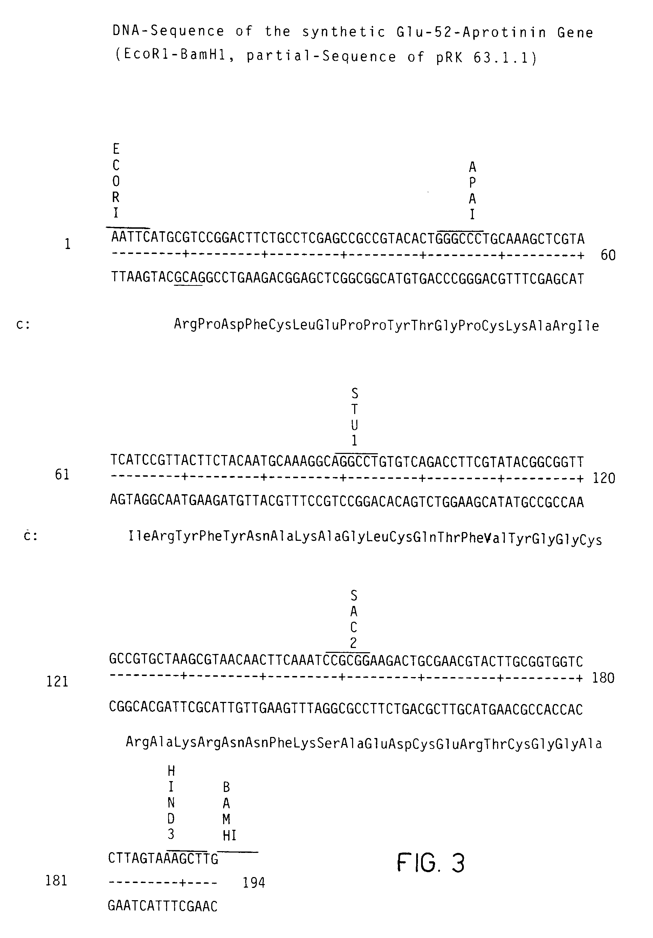

Die synthetischen Gene für Aprotinin und Aprotinin-Homologe wurden über ein Master-Gen durch das Zusammenfügen von 15 gereinigten Oligonukleotiden, die überlappende terminale Bereiche besitzen (s. Fig. 1) konstruiert. Diese Konstruktion erfolgte in zwei Schritten. 1. Teil A und Teil B des Gens wurde durch Hybridisierung hergestellt, über Ligierung und Reinigung der DNA-Fragmente 1, 2, 3, 4 und 6 Teil A und der DNA-Fragmente 5, 7, 8, 9, 10, 11, 12, 13, 14 und 16 Teil B (Fig. 2). 2. Teil A und Teil B des Gens wurden zusammen ligiert und gereinigt. Für diese Konstruktion wurden Materialien eingesetzt und Verfahren angewendet, wie sie im folgenden beschrieben werden. Die DNA-Sequenz des Master-Gens ist in Fig. 3 wiedergegeben. Sie umfaßt das Initiationscodon ATG, zwei Terminierungscodons, TAG und TAA, am 5'-Terminus die Restriktionsstelle für Eco RI und an 3'-Terminus die Restriktionsstelle für Hind III und Bam HI, sowie die internen Restriktionsstellen für Apa I, Stu I, Sac II (Sst II). Diese Stellen, insbesondere die internen, erleichtern das Klonieren der kodierenden Sequenz, die Modifizierung des Master-Gens durch Austausch von DNA-Fragmenten, die für andere Aminosäuren kodieren, oder die einen anderen Codongebrauch haben. Die Amplifizierung der Gene kann in einfacher Weise durch Zufügen geeigneter Linkersequenzen erfolgen. Das Spektrum der Bearbeitung durch Protein Engineering wird durch eine solche Konstruktion ermöglicht.

Um Gene für Aprotinin-Homologe zu konstruieren, muß lediglich ein Restriktionsfragment durch die geeignete DNA-Sequenz ausgetauscht werden. Sequenzen für solche Fragmente, die für Aminosäureaustausche in den Positionen 15 und 52 kodieren, werden in Fig. 2b gezeigt.

Die rekombinanten Plasmide wurden wie folgt konstruiert:

Das für die experimentelle Aprotininklonierung gewählte Plasmid war pUC 8 (J.Vieira und J. Messing, 1982 Gene, 19, 259). Dieser Klonierungsvektor besteht aus einem von pBR 322 abgeleiteten Ampicillin-Resistenzgen und dem "ori" der DNA-Replikation, ligiert an einen Abschnitt des lac-Z-Gens, welches eine Reihe von einmal vorkommenden Erkennungsstellen für Restriktionsenzyme enthält. Wenn dieser Vektor in geeignete lac⁻ E. coli-Zelten eingeführt wird, so bilden die Transformanten auf geeigneten Indikatorplatten blaue Kolonien. Das Klonieren von DNA-Fragmenten in jede der multiplen Restriktionsstellen, z.B. zwischen Eco RI und Bam HI, inaktiviert das lac-Gen, was zur Bildung von weißen Kolonien führt. Plasmid pUC 8 ist im Handel von P-L Biochemicals erhältlich.

4

Die Expressionsplasmide wurden wie folgt konstruiert:

Zur Expression von Aprotinin und Aprotinin-Homologen als Fusionsprotein wurde ein Plasmid konstruiert, in welchem das geeignete Gen mit dem Carboxyterminus des $\beta$-Galactosidase-Gens fusioniert wurde, wie dies in ähnlichen Experimenten von U. Rüther und B. Müller-Hill 1983, EMBO Journal, 2, Seiten 1791-1794 gezeigt wurde. Das "Eltern"-Plasmid pUR 278 hat die einmal vorkommenden Klonierungsstellen: Bam HI, Sal I, Pst I, Xba I und Hind III am 3'-Ende des lac-Z-Gens (siehe auch DE-OS 33 09 501.9). Die Insertion einer kodierenden DNA-Sequenz in die geeigneten Klonierungsstellen und im richtigen Leserahmen führt zu einem Fusionsprotein von aktiver $\beta$-Galactosidase in Kombination mit dem Peptid, für das die DNA kodiert.

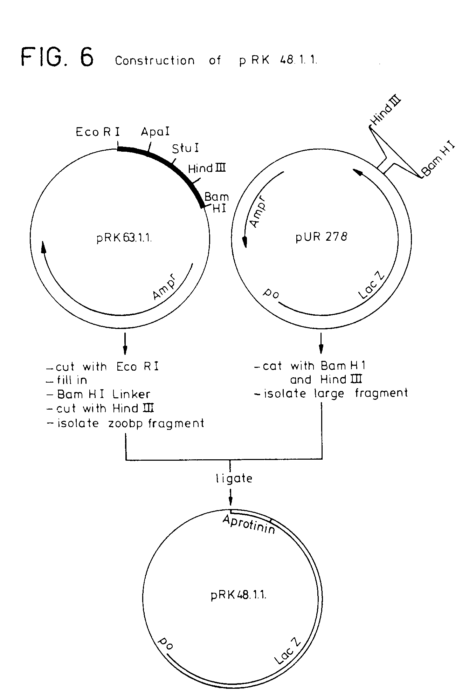

Die Restriktionsstellen Bam HI und Hind III des Expressionsvektors pUR 278 wurden zur Klonierung der synthetischen Gene für Aprotinin und Aprotinin-Homologe in einem Expressionsvektor ausgewählt. Es war deshalb erforderlich, das Aprotiningen durch Zufügen einer Bam HI-Stelle am 5'-Eco R$^l$-Ende des Gens und unter Verwendung der Hind III-Stelle am 3'-Ende zu modifizieren (s. auch Fig. 6).

Die folgenden Standardmaterialien und Methoden wurden für die rekombinante DNA-Arbeit eingesetzt:

Die hier beschriebenen synthetischen Gene, rekombinanten Plasmide und Expressionsvektoren mit solchen Genen können durch folgende Materialien und Methoden hergestellt und charakterisiert werden.

### Material

### Enzyme

Polynukleotid-Kinase (PNK), 5,5 Einheiten/$\mu$l; Boehringer-Mannheim Nr. 633 542;

DNA-Polymerase; Klenow, Boehringer-Mannheim Nr. 104 523; T4-DNA-Ligase, 0,9 Einheiten/$\mu$l; Boehringer-Mannheim Nr. 481 220;

Restriktionsenzyme wurden von Bethesda Research Labs, Boehringer Mannheim, Biolabs, erworben.

Alkalische Phosphatase aus Kälberdarm (CIP); Boehringer-Mannheim;

Lysozym; RNase A; Boehringer Mannheim.

### Reagentien

Gamma 32 P ATP; Amersham Nr. PB 10168

Alpha 32 P - dTTP; Amersham 167

ATP; Sigma Nr. A-6144

Bis-Acrylamid; Serva 29195

Acrylamid; Serva und Bio-Rad 161-0103

TEMED; Serva 35925

Ammonium-persulfat; Serva 13375

Harnstoff; BRL ultra-rein 5505 UA

DE 52 (vorgequollene Diethylaminoethyl-cellulose); Whatman, Cat. 4057-050

DTE; Serva 20697

Isopropyl-$\beta$-D-thiogalactosid (IPTG); Sigma I 5502

5-Brom-4-chlor-indolyl-$\beta$-D-galactosid (X gal) ; Boehringer 651745

N,N'Dimethylformamid; Merck 2 203 034

EGTA

Saccharose; BRL 5503 UA

Diaminopimelinsäure; Sigma D 1377

M 13 - Didesoxynucleotid-Sequenzierungssystem; New England

Biolabs, Beverly, MA USA # 408, # 409

Chloroform

Isoamylalkohol

Thymidin; Serva 18600

Glucose D ( + ); Merck 8337

Tris; Merck 8382

Kaliumhydroxid; Merck 5033

Kalziumchlorid; Merck 2382

Rubidiumchlorid; Sigma R 2252

Manganchlorid; Sigma M 3634

DMSO, Sigma D 5879

EDTA;

Kaliumacetat;
SDS;

DNA

Plasmid pUC 8; Pharmacia P-L Biochemicals 27-4916-xx
Bam HI-Linker

Medien und Antibiotika

Bacto-Trypton; Difco 0123-01
Bacto-Hefe-Extrakt; Difco 0127-01
Bacto-Agar; 0140-01

| LB-Medium: | (für 1 l) 10 g Bacto-Trypton, 4 g Bacto-Hefeextrakt, 10 g NaCl, eingestellt auf pH 7,5 mit NaOH |
|---|---|
| kappa 1776-Medium: | (für 1 l) 25 g Bacto-Trypton, 7,5 g Bacto-Hefe-Extrakt, 1M Tris-HCl (pH 7,5) 20 ml, Zugabe von 950 ml, autoklaviert und abgekühlt, Zugabe von sterilem: 5 ml 1M $MgCl_2$, 10 ml 1 %ige Diaminopimelinsäure, 10 ml 0,4 %iges Thymidin, 25 ml 20 %ige Glukose |
| YT-Medium: | (für 1 l) 8 g Bacto-Trypton, 5 g Bacto-Hefe-Extrakt, 5 g NaCl |

Agarplatten wurden hergestellt durch Zugabe von 15 g
Bacto-Agar zu 1 l des geeigneten Mediums.

Indikatorplatten: Zu 1 l im Autoklaven behandeltem YT-Medium mit 1,5 % Agar wurden die folgenden Lösungen zugegeben: 2 ml 0,1 M IPTG, 2 ml 2 %iges X-gal in N,N'-Dimethylformamid und 2 ml 100 mg/ml Ampicillin.

Antibiotika

Chloramphenicol; Boehringer Mannheim 634 433
Ampicillin; Serva 13397
Tetracyclin; Serva 35865

Puffer und Lösungen

| 20 µM ATP | in Wasser |
|---|---|
| 10 mM ATP | in Wasser |
| 10X PNK-Mix: | 0,5 M Tris-HCl (pH 7,6); 0,1 M $MgCl_2$; 50 mM DTE; 1 mM EDTA |
| 10X Ligase-Mix: | 0,5 M Tris-HCl (pH 7,4); 0,1 M $MgCl_2$; 0,1 M DTE; 10 mM ATP 10X SP-50: 100 mM Tris-HCl (pH 7,5), 100 mM $MgCl_2$; 500 mM NaCl; 10 mM DTT |
| 10X SP-100: | 100 mM Tris-HCl (pH 7,5); 100 mM $MgCl_2$; 1 M NaCl; 10 mM DTT |
| 10 SP-O: | 100 mM Tris-HCl (pH 7,5); 100 mM $MgCl_2$; 10 mM DTT |
| 1 M TBE: | 1 M Tris; 0,83 M Borsäure; 10 mM EDTA; pH 8,3 |
| 3X Formamid-Farbstoff-Mix: | 70 % Formamid; 20 % Glycerin; 1 mM EDTA; 0,33 mg/ml Bromphenolblau; 0,66 mg/ml Xylencyanol FE; 0,66 mg/ml Orange G |
| 20X E-Puffer: | 0,8 M Tris; 0,4 M Natriumacetat; 40 mM EDTA; pH 8,3 |
| 10X CIP-Puffer: | 0,5 M Tris-HCl (pH 9,0), 10 mM $MgCl_2$, 1 mM $ZnCl_2$, 10 mM Spermidin |
| Transformationspuffer: | hergestellt wie folgt: 15 g Saccharose, 1 ml 3,5 M KOH, 1 ml 1 M $CaCl_2$, 2 ml 5,0 m RbCl, werden mit Aqua bidest auf 50 ml gebracht, mit 10 %iger Essigsäure auf pH 6,2 eingestellt, 1 ml 4,5 M $MnCl_2$ zugegeben, mit 10 %iger Essigsäure auf pH 5,8 eingestellt, mit Aqua bidest auf 100 ml aufgefüllt und steril filtriert. |
| TE-Puffer: | 10 mM Tris-HCl pH 8,0, 0,1 mM EDTA |
| Lysozym-Mix: | 50 mM Glukose, 1 mM EDTA, 10 mM Tris-HCl, pH 8,0 |
| Phenol/Sevag: | Gemisch aus 1 Volumen 80 %igem Phenol und 1 Volumen Sevag (Chloroform: Iso-Amylalkohol, 24:1) |

Standardmethoden

Standardmethoden der rekombinanten DNA-Technik wurden nach Maniatis et al, 1982, in Molecular Cloning, Cold Spring Harbor Laboratory, Cold Spring Harbor, USA, mit einigen Modifizierungen, die nachfolgend beschrieben werden, angewendet.

Standard-Ethanol-Fällung

DNA-Pellets wurden gelöst oder Lösungen wurden auf 0,3 M Natriumacetat eingestellt, zwei Volumenteile Ethanol wurden zugegeben, 15 min bei -70°C inkubiert und zentrifugiert. Die Pellets wurden zweimal mit 80 %igem Ethanol gewaschen und im Vakuum getrocknet.

Standard-Phenol-Extraktion

Die Lösungen wurden gründlich mit Phenol/Sevag, Volumenverhältnis 1:1 vermischt, zentrifugiert, die Phenolphase wurde mit 1/10 Volumen Puffer oder Wasser reextrahiert, die wäßrigen Phasen wurden vereinigt.

Standard-Isolierung von DNA-Fragmenten nach Polyacrylamid-Gel-Elektrophorese

Kleine DNA-Fragmente (bis zu 250 Nukleotiden) wurden durch Gelelektrophorese abgetrennt. Die Banden wurden durch Autoradiographie oder UV-Licht sichtbar gemacht (vorbeschichtete TLC-Platten Silgur-25,Macherey-Nagel & Co.). Die Banden wurden ausgeschnitten, mit einem silikonisierten Glasstab homogenisiert, mit ca. 400 $\mu$l TE-Puffer, pH 8,0, bei 42°C 18 h eluiert. Das Material wurde zentrifugiert und die Pellets wurden 4 h bei 42°C reeluiert und zentrifugiert. Beide Überstände wurden vereinigt und durch Anionenaustauschchromatographie auf kleinen DE-52 Pasteur-Pipettensäulen mit 1M TEABC-Puffer gereinigt. Nach dem Lyophilisieren wurde die DNA zweimal in Wasser aufgelöst und lyophilisiert.

Standard-Ligierung

Zur Standard-Ligierung (Fragment kleiner als Vektor) wurde ein molares Verhältnis von 1:5 für Vektor:Fragment angewendet. Die DNA-Endkonzentration betrug 25 $\mu$g/ml. Die DNA wurde in einer kleinen Menge TE-Puffer gelöst, 10X-Ligase-Mix, T4 DNA-Ligase wurde zugegeben und eingestellt auf 1X Ligase-Mix-Konzentration (50 mM Tris-HCl, pH 7,4, 10 mM MgCl$_2$, 10 mM DTE, 1 mM ATP; Standardvolumen 30 $\mu$l). Die Reaktion erfolgte bei 14°C für 16 h.

Standard-5'-Markierung von DNA-Fragmenten

Desphosphorylierte DNA (Endkonzentration ca. 0,2 $\mu$M) wurde in 1X Kinase-Puffer I (50 mM Tris-HCl, pH 7,6, 10 mM MgCl$_2$, 5 mM DTE, 0,1 mM EDTA) aufgelöst. Zusammen mit nichtmarkiertem ATP wurde Gamma 32 P ATP (3000 Ci/mMol) zugegeben. Die Endkonzentration an ATP war stets größer als 1 $\mu$M. Die Reaktion wurde mit einem 500 - 1000-fachen Überschuß an Polynukleotidkinase, berechnet im Hinblick auf die Einheitsdefinition und die DNA-Konzentration) 30 min bei 37°C durchgeführt. Die Reaktion wurde zur Phenolextraktion gestoppt. DNA wurde mit Ethanol ausgefällt, gewaschen und getrocknet.

Standard-Abbau mit Restriktionsendonuklease

Die Verdauung mit Restriktionsendonukleasen wurde in der Hauptsache gemäß den Angaben der Hersteller durchgeführt. Gereinigte, salzfreie DNA wurde im Puffer (SP-0, SP-50 oder SP-100 entsprechend dem verwendeten Enzym) gelöst und mit einer geeigneten Enzymmenge verdaut. Schließlich wurde das Material mit Phenol extrahiert und mit Ethanol ausgefällt.

Standardisolierung von DNA-Fragmenten nach Agarose-Gel-Elektrophorese

DNA-Fragmente wurden durch Agarose-Gel-Elektrophorese aufgetrennt (s. T. Maniatis et al, 1982, Cold Spring Harbor Laboratory, Molecular Cloning), mit Ethidiumbromid angefärbt und unter einem langwelligem UV-Licht ausgeschnitten. Die Agarscheiben wurden in einem Dialysierschlauch gegeben, mit 0,5X E-Puffer (Volumenverhältnis Puffer: Agarscheibe wie 1,5:1) gefüllt; sie müssen vom Puffer gut umgeben sein. Der verschlossene Schlauch, der frei von Luftblasen war, wurde in eine Elektrophoresekammer gegeben, die mit 0,5X E-Puffer gefüllt war. Die Elektrophorese wurde 30 min bei 200 V ausgeführt, dann wurde der Strom 30 sekundenlang umgepolt, um die DNA von der Wand des Dialysierschlauches freizusetzen. Der Puffer, der die Agarscheibe umgab, wurde sorgfältig entfernt und die DNA wurde über DEAE-Cellulose oder DE 52-Säulen (siehe oben) gereinigt.

## Standard-Desphosphorylierung von DNA

Vollständig geschnittene und gereinigte DNA wurde in Wasser aufgelöst und auf 1X CIP-Puffer (Standard-Gesamtvolumen 48 $\mu$l) eingestellt. Die Reaktion wurde bei 37°C durch Zugabe von 1 $\mu$l (20 Einheiten) Phosphatase aus Kälberdarm (CIP) gestartet; nach 30 min wurde wiederum 1 $\mu$l CIP zugegeben. Die Reaktion wurde nach 1 h durch Zugabe von 5 $\mu$l 50 mM EGTA und 10-minütige Inkubation bei 65°C gestoppt. Zur Dephosphorylierung von DNA mit glatten Enden oder versetzten 5'-Enden wurden wiederholt Inkubationen für jeweils 15 min bei 37°C und für 15 min bei 56°C durchgeführt. Die DNA wurde mit Phenol/Sevag extrahiert und mit Ethanol ausgefällt.

## Autoradiographie

Filme: AGFA-Gevaert, Curix RP 1, 100 AFW, Kodak XAR 5, 165 1512 Röntgenfilmentwickler, AGFA G153, Kodak LX24
Röntgenfilmfixierer; AGFA G353; Kodak AL4.

## Standard-Transformationsverfahren

Transformationen wurden gemäß dem Verfahren von D. Hanahan (1983, J. Mol, Biol., 166, 557-580) durchgeführt.
1 ml einer 20 ml Übernacht-Kultur des Wirtstammes, die mit einer einzigen Kolonie inokuliert und in kappa 1776 Medium (37°C, Schüttler mit 200 Upm) vermehrt wurde, diente zur Inokulierung von 100 ml vorgewärmtem (37°C) kappa 1776 Medium.
Diese Kultur wurde unter den gleichen Bedingungen vermehrt. Das Zellwachstum wurde bei 0,2 OD 500 nm gestoppt. Nach Abkühlen auf 4°C und Zentrifugation wurde das Zellpellet in 20 ml eiskalten Transformationspuffer resuspendiert und 5 min bei 0°C inkubiert. Die Suspension wurde wiederum zentrifugiert (3000 Upm, 4°C, 15 min) und in 4 ml eiskaltem Transformationspuffer resuspendiert. Nach Zugabe von 7 $\mu$l DMSO 200 $\mu$l aliquote Teile, wurden die Zellen weitere 15 bis 60 min in Eiswasser inkubiert. Zu einem solchen aliquoten Teil kompetenter Zellen wurde DNA, aufgelöst in 20 $\mu$l TE, zugegeben und das Gemisch 20 min in Eiswasser und dann 3 min bei 42°C inkubiert. 1 ml vorgewärmtes (37°C) kappa 1776-Medium wurde mit einem solchen Aliquot inokuliert und die Kultivierung 1 h bei 37°C ausgeführt. Zum Ausstreichen der Transformanten wurden die Zellen zentrifugiert (3000 Upm, 15 min, 4°C), im YT-Medium resuspendiert und auf Indikatorplatten ausgestrichen. Entsprechend der erwarteten Anzahl an Transformanten wurde eine gewisse Menge der Suspension zum Ausstreichen verwendet.

## Schnelle analytische Standardplasmidisolierung

Dieses Verfahren ist eine Modifizierung der Methode von Birnboim und Doly, 1979 (siehe auch T. Maniatis et al, 1982). Von jedem zu analysierenden Transformanten wurde über Nacht eine 2 ml Kultur angezogen (hölzerner Zahnstocher, 37°C, 16 h, rotierendes Rad). 1,5 ml der Übernacht-Kultur wurden 1 min bei 12000 g zentrifugiert (Eppendorf-Zentrifuge). Das Pellet wurde in frisch bereiteter Lösung aufgelöst (2 mg Lysozym pro ml Lysozym-Mix), und dann 5 min bei 20°C inkubiert. Die Probe wurde 5 min nach Zugabe von frisch hergestellter eiskalter 0,2 M NaOH, welche 1 % SDS enthält in Eis inkubiert. Zur Ausfällung chromosomaler DNA und der Proteine wurden 150 $\mu$l eiskaltes Kaliumacetat, pH 4,8, zugegeben. Nach 5-minütiger Inkubation bei 0°C und 10-minütiger Zentrifugation bei 12000 g wurde der plasmidhaltige Überstand in ein frisches Eppendorf Hütchen transferiert und mit Chloroform/Isoamylalkohol (24:1) extrahiert. 500 $\mu$l Isopropanol wurden zu der wäßrigen Phase zugegeben. Das Gemisch wurde 30 min bei -20°C inkubiert. Nach Zentrifugation (10 min, 12000 g) wurde das Sediment mit 80 %igem Ethanol gewaschen und kurz im Vakuum getrocknet. Das Material reicht für 5 bis 6 verschiedene Restriktionsanalysen, die mit Hilfe von Gelektrophoresen ausgeführt werden.

Standardreinigung von Oligonukleotiden durch Polyacrylamid-Gelelektrophorese

Oligonukleotide (ca. 20 OD 260 nm) wurden in gepuffertem Formamid (0,1 M TBE) aufgelöst und elektrophoretisch in 7 M Harnstoff, 20 % Polyacrylamid-Gelen aufgetrennt (Maxam und Gilbert, Meth. Enzymol. 65, 500-560, 1980). Die Gele wurden auf Fluoreszenz-Dünnschichtplatten gelegt; die DNA wurde mit UV-Licht sichtbar gemacht. Isolierung und Reinigung erfolgten, wie dies im Standardprotokoll zur Isolierung von DNA nach Acrylamid-Gelelektrophorese angegeben ist (s. oben). Die Qualität dieses Verfahrens wurde routinemäßig durch analytische 5'-Phosphorylierung mit gamma 32 P ATP und Polyacrylamid-Gelelektrophorese überprüft.

Herstellung von Glu-52-Aprotinin aus β-Galactosidase-Lys-15-Glu-52-Aprotinin-Fusionsprotein

Viele Proteinen, die in großen Mengen in Bakterien synthetisiert werden, akkumulieren sich in unlöslicher Form (D.C. Williams, R.M. Van Frank, J.B. Burnett, W.L. Muth, 1982, Science 215, 687). Diese unlöslichen Proteine werden als Einschlußkörper (inclusion bodies) bezeichnet. Sie können leicht von anderen Zellproteinen abgetrennt und gewöhnlich nur unter denaturierenden Bedingungen solubilisiert werden.

Der E. coli Stamm RR 1ΔM 15 (ATCC 35 102) wurde mit dem Plasmid pRK 48.1.1. transformiert, welches für das Glu-52-Aprotinin-β-Galactosidasegen das hinter einem E. coli-Promoter, Operator und einer Ribosomenbindungsstelle liegt, kodiert. Die maximale Ausbeute von Glu-52-Aprotinin-β-Galactosidase-Fusionsprotein betrug 20 % bezogen auf das gesamte Zellprotein. Die Einschlußkörper akkumulieren sich im allgemeinen an den polaren Bereichen, wobei ein großer Prozentsatz von Zellen, normaler Länge, Einschlußkörper in der Nähe eines jeden Pols aufweist.

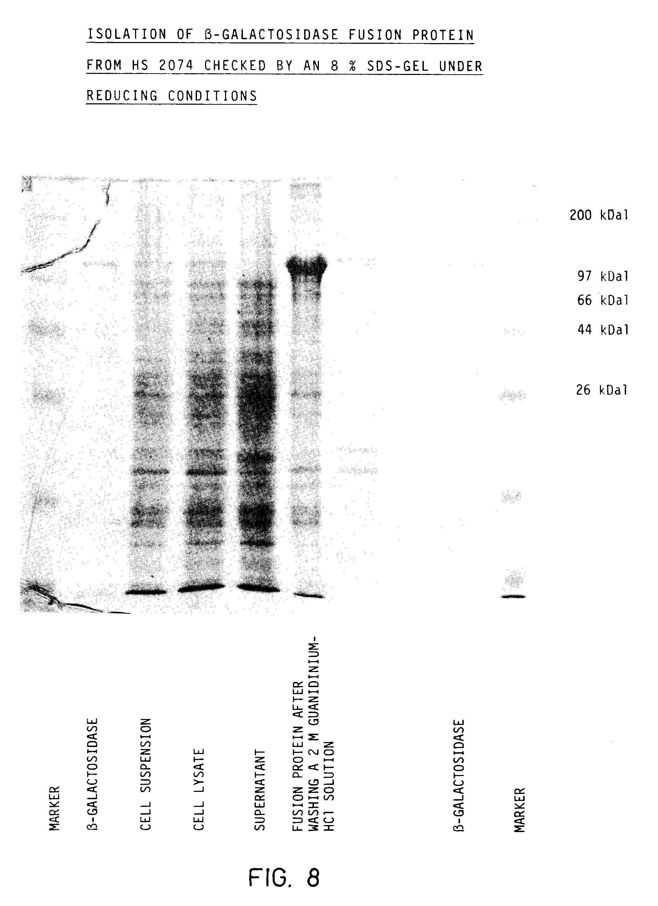

Eine Obernachtkultur von E. coli-Stamm RR1 Δ M15 wurde zentrifugiert, und das Pellet wurde dann in einem Puffer zum Aufschließen resuspendiert. Nach Ultraschall-Behandlung wurde das Zellysat zur Gewinnung der Einschlußkörper zentrifugiert. Die Einschlußkörper wurden mit 2 M Guanidinium-Hydrochlorid gewaschen. Die Reinigungsschritte wurden mittels SDS-Polyacrylamid-Elektrophorese nach Laemmli (U. K. Laemmli 1970, Nature 277, 680-685) Fig. 8, überprüft.

Zur Gewinnung von intaktem Lys-15-Glu-52-Aprotinin müssen die Einschlußkörper solubilisiert und durch Bromcyan gespalten werden. Anschließend muß Glu-52-Aprotinin in die korrekte räumliche Struktur überführt werden.

Die Einschlußkörper konnten in 6M Guanidinium-hydrochlorid, welches eine ausreichende Menge an DTT enthielt, solubilisiert werden. Nach Abtrennung unlöslicher Teile wurde das Fusionsprotein durch Dialyse gegen Wasser, das 10 mM Mercaptoethanol enthielt, ausgefällt. Das feuchte Fusionsprotein wurde in 70 %iger Ameisensäure aufgelöst und mit Bromcyan nach Gross (E. Gross, B. Witkop 1961. Amer. Chem. Soc. 83, 1510-1511) gespalten.

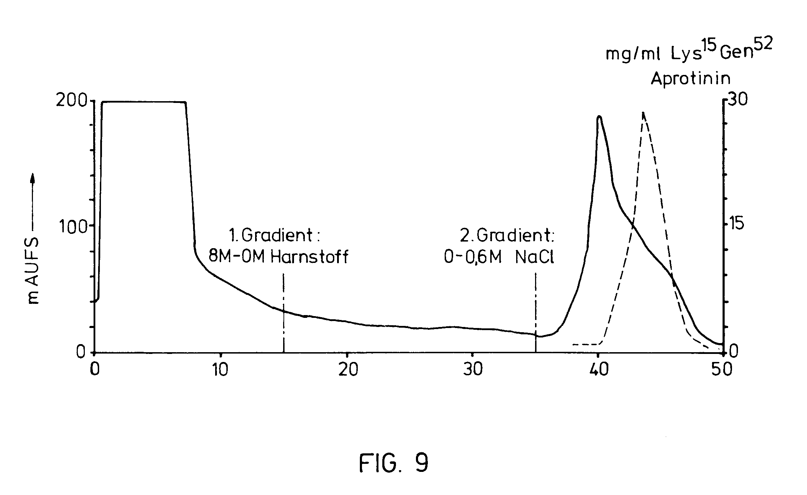

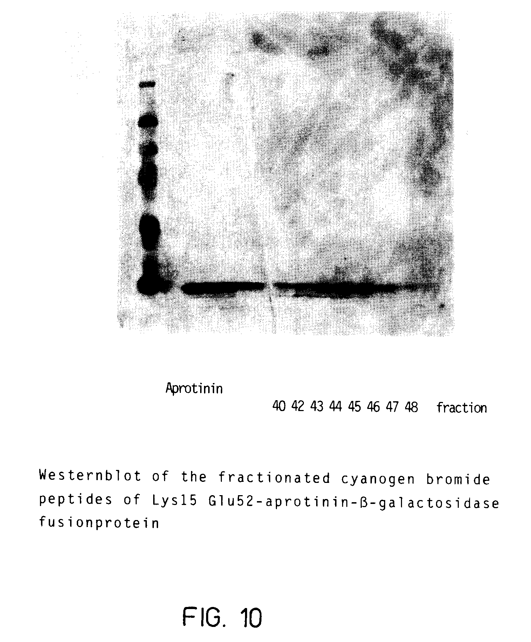

Das Glu-52-Aprotinin wurde von den Bromcyanfragmenten der β-Galactosidase durch Ionenaustauschchromatographie abgetrennt und gleichzeitig nach dem Verfahren von Creighton (T.E. Creighton, Proceedings of Genex-UCLA Symposium 1985, Ingstone; in Druck (Fig. 9) renaturiert. Der aktive Inhibitor konnte durch Western-Blot Analyse (Fig. 10) nachgewiesen werden.

Die aktiven Fraktionen wurden durch Eindampfen konzentriert und dann gegen 0,1 M NH$_4$HCO$_3$ dialysiert. Nach Lyophilisation wurde der Inhibitor durch HPLC auf einer "wide-pore" RP-18-Säule gereinigt, wobei ein Gradient aus o,1 % TFA in Puffer A und 0,1 % TfA 60 % CH$_3$CN in Puffer B verwendet wurde.

Die aktiven Fraktionen wurden vereinigt,und der Inhibitor wurde durch Mikrosequenzierung mit einem Gasphasensequenator gemäß Hewick (R.M. Hewick, M.W. Hunkapiller, L.E. Hood, W.I. Dreyer 1981, J. Biol. Chem. 256, 7990-7997) charakterisiert. Die ersten 20 Reste am N-Terminus sind mit denen des erwarteten Inhibitors identisch (Tabelle 2). Die Aminosäureanalyse zeigt, daß der Inhibitor die erwartete Aminosäurezusammensetzung aufweist (Tabelle 1).

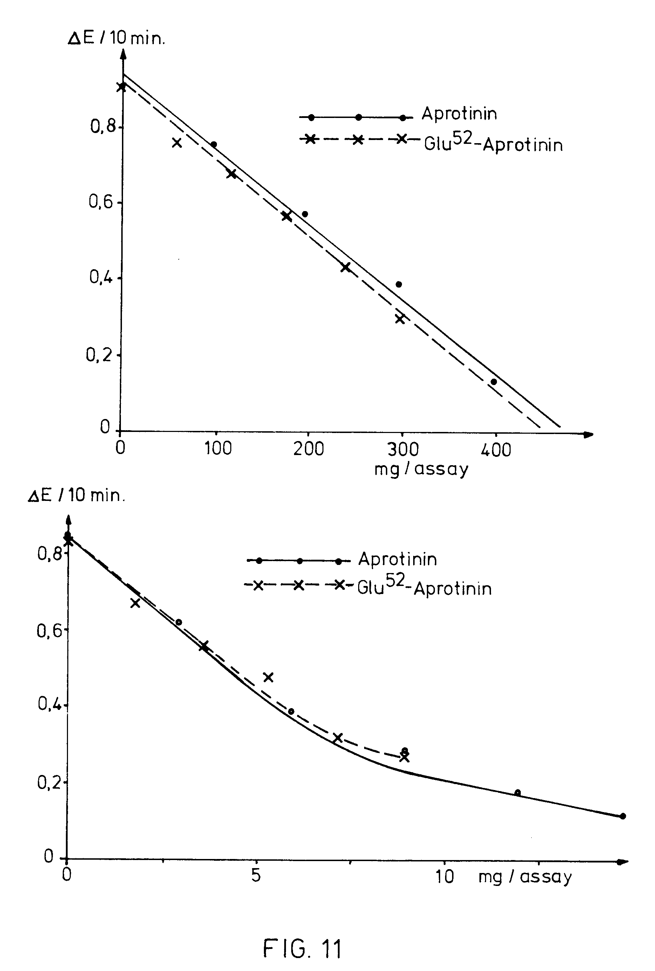

Ein Vergleich von Aprotinin und Glu-52-Aprotinin in Bezug auf ihre Trypsin-inhibierende Wirkung ergibt identische Kurven in Abhängigkeit von der Konzentration (Fig. 11).

Alle diese Experimente zeigen, daß es möglich ist, Glu-52-Aprotinin als ein Fusionsprotein in E. coli herzustellen und es nach Spaltung und Trennung unter renaturierenden Bedingungen zu isolieren.

Herstellung von Val-15-Glu-52-Aprotinin und anderen Aprotinin-Derivaten

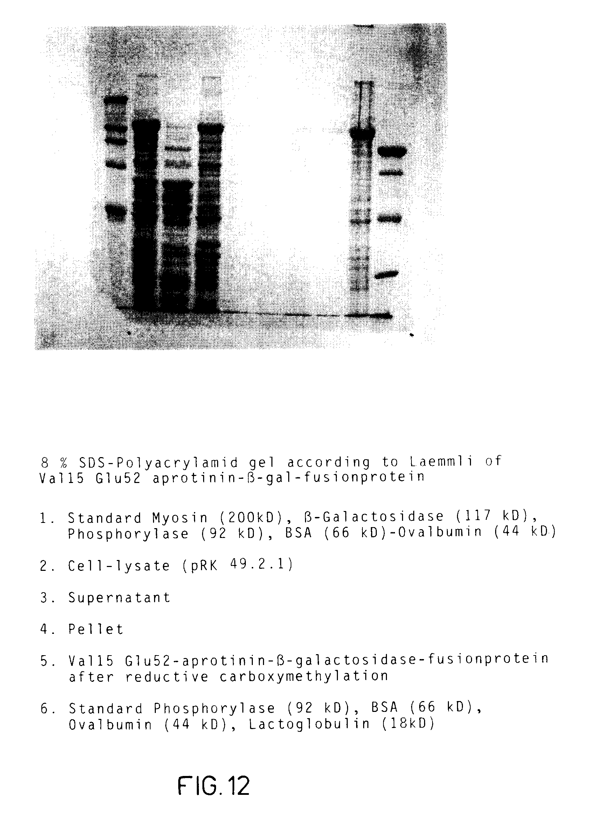

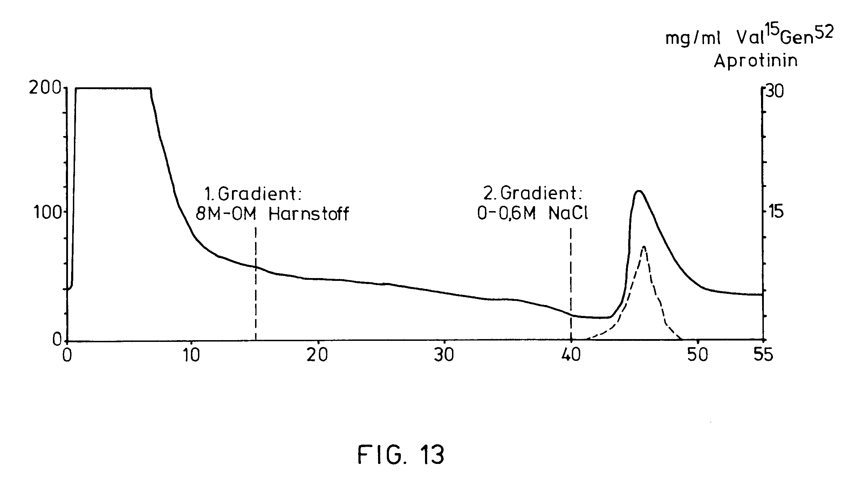

Val-15-Glu-52-Aprotinin kann in ähnlicher Weise aus β-Galactosidase-Fusionsprotein hergestellt werden, wie dies für Glu-52-Aprotinin beschrieben ist (Fig. 12, 13). Es wird mit Hilfe eines Elastase-Hemmtests gemessen.

Der Inhibitor wurde durch Aminosäureanalyse und N-terminales Sequenzieren charakterisiert (Tabellen 1 und 2).

Alle anderen Aprotinin-Derivate konnten in ähnlicher Weise hergestellt werden, wie dies für Glu-52-Aprotinin und Val-15-Glu-52-Aprotinin beschrieben ist.

Tabelle 1

| Aminosäureanalyse von Aprotinin, Glu-52-Aprotinin und Val-15-Glu-52-Aprotinin | | | |
|---|---|---|---|
| Aminosäure | Aprotinin | Glu-52 | Val-15-Glu- |
| Asp | 4,75 (5) | 4,92 (5) | 5,10 (5) |
| Thr | 2,90 (3) | 2,91 (3) | 2,85 (3) |
| Ser | 0,98 (1) | 1,01 (1) | 0,95 (1) |
| Glu | 2,90 (3) | 4,30 (4) | 4,30 (4) |
| Gly | 5,92 (6) | 5,91 (6) | 6,30 (6) |
| Ala | 6,00 (6) | 6,00 (6) | 6,00 (6) |
| Val | 1,04 (1) | 1,02 (1) | 2,06 (2) |
| Met | 0,95 (1) | - | - |
| Ile | 1,29 (2) | 1,30 (2) | 1,35 (2) |
| Leu | 2,10 (2) | 2,01 (2) | 2,01 (2) |
| Tyr | 3,92 (4) | 3,70 (4) | 3,81 (4) |
| Phe | 3,86 (4) | 4,08 (4) | 4,05 (4) |
| Lys | 3,99 (4) | 3,80 (4) | 3,10 (3) |
| Arg | 5,82 (6) | 5,75 (6) | 6,00 (6) |

Die Aminosäuren wurden bestimmt nach post-Säulen-Derivatisierung mit o-Phthalaldehyd. Cys und Pro wurden nicht bestimmt.

Tabelle 2: Aminosäuresequenzierung von Glu-52- und Val-15-Glu-52-Aprotinin (N-terminales Ende)

1. Glu-52-Aprotinin; ca. 1 nMol der Substanz wurde über 20 Zyklen sequenziert:

1                                                              14
Arg-Pro-Asp-Phe-Cys-Leu-Glu-Pro-Pro-Tyr-Thr-Gly-Pro-Cys-

15                      20
Lys-Ala-Arg-Ile-Ile-Arg-

2. Val-15-Glu-52-Aprotinin; ca. 1 nMol der Substanz wurde über 20 Zyklen sequenziert:

1                                                              14
Arg-Pro-Asp-Phe-Cys-Leu-Glu-Pro-Pro-Tyr-Thr-Gly-Pro-Cys-

15                      20
Val-Ala-Arg-Ile-Ile-Arg-

Vergleich Aprotinin/Glu-52-Aprotinin

Glu-52-Aprotinin, das nach Spaltung mit BrCN erhalten wurde, wurde mit authentischem Aprotinin im Hinblick auf seine inhibierende Wirkung gegen Schweine-Trypsin verglichen. Die beiden Substrate Pyrglu-Gly-Arg-pNA und Benzoyl-Arg-pNA wurden zur Trypsinbestimmung verwendet.

Die Stammlösung für Aprotinin enthielt 1 $\mu$g/ml, bei Glu-52-Aprotinin 0,6 $\mu$g/ml. Von diesen Stammlösungen wurden 0 - 100 - 200 - 300 - 400 - 500 $\mu$l beim Test mit Benzoyl-Arg-pNA verwendet. Beim Test mit Pyrglu-Gly-Arg-pNA wurden 0 - 3 - 6 - 9 - 12 - 15 $\mu$l verwendet. Die Ergebnisse sind in Tabelle 3 zusammengestellt.

Tabelle 3

| Substrat | Aprotinin | | Glu-52-Aprotinin | |
|---|---|---|---|---|
| | Inhibitormenge pro Assay | $\Delta$E/10 min | Inhibitormenge pro Assay | $\Delta$E/10 min |
| Benzoyl-Arg-pNA | 0 ng | 0,91 | 0 ng | 0,91 |
| | 100 | 0,76 | 60 | 0,77 |
| | 200 | 0,57 | 120 | 0,68 |
| | 300 | 0,39 | 180 | 0,58 |
| | 400 | 0,08 | 240 | 0,44 |
| | 500 | 0,00 | 300 | 0,30 |
| Pyr-Glu-Gly-Arg-pNA | 0 ng | 0,85 | 0 ng | 0,84 |
| | 3 | 0,62 | 1,8 | 0,67 |
| | 6 | 0,39 | 3,6 | 0,56 |
| | 9 | 0,29 | 5,4 | 0,47 |
| | 12 | 0,18 | 7,2 | 0,32 |
| | 15 | 0,13 | 9,0 | 0,28 |

Diese Ergebnisse zeigen, daß Aprotinin und Glu-52-Aprotinin sich in beiden Trypsin-Inhibitor- Assays identisch verhalten. Dies zeigt nicht nur, daß Glu-52-Aprotinin praktisch zu 100 % aktive Moleküle enthält, sondern auch daß die Gleichgewichtskonstanten der Trypsin-Inhibitor-Komplexe die gleiche Größenordnung besitzen.

Western-Blot

Es wurde ein Western-Blot durchgeführt, wie dies von Towbin (H. Towbin, T. Staehelin), I. Gordon 1979, Proc. Natl. Acad. Sci. USA 76, 4350-4354) beschrieben ist. Für den Nitrocellulose-Blot dienten polyklonale Anti-Aprotinin-Antikörper von Kaninchen als primäre und biotinylierte Esel-Anti-Kaninchen-Antikörper als sekundäre Antikörper. Innunkomplexe wurden unter Verwendung unter Verwendung eines Streptavidin-Biotin-Merrettichperoxidase-Komplexes mit 4-Chlor-1-naphthol als Substrat, wie dies im Manual des Herstellers beschrieben ist (AMERSHAM BUCHLER, Braunschweig), nachgewiesen.

ELISA

Ein Festphasen-Enzym-Immunosorbent-Assay (ELISA) wurde als kompetitiver Assay unter Verwendung von Mikrotiter-Antikörper-Platten durchgeführt, wie dies von Müller-Esterl (W. Müller-Esterl, A. Oettl, E. Truscheit, H. Fritz, Fresenius Z. Anal. Chem. (1984) 317, 718-719) beschrieben ist.

Bestimmung der Aminosäuresequenz

Ca. 0,5 bis 2 nMol des Proteins wurden in 30 $\mu$l TFA gelöst. Die Probe wurde auf ein Glasfaserfilter gegeben, das mit 3 mg Polybren vorbehandelt war. Die Sequenzanalyse erfolgte mit Hilfe des Gasphasen-Proteinsequenators von APPLIED BIOSYSTEMS (Inc. USA) nach Hewick (R. M. Hewick, M.W. Hunkapiller, L.E. Hood, W. Dreger 1981, J. Biol. Chem. 256, 7990-7997). Die schrittweise freigesetzten Aminosäure-Phenylthiohydantoin-Derivate wurden unter Verwendung einer Cyano-HPLC-Säule (DU PONT) und eines Trennsystems, wie es von Beyreuther (K. Beyreuther, B. Biesler, J. Bowens, R. Dildrop, K. Neufer, K. Stüber, S. Zais, R. Ehring, P. Zabel 1983, Modern Methods in Protein Chemistry S. 303-325, Walter de Gruyter & Co., Berling) beschrieben ist, analysiert. Dabei wurde ein WATERS HPLC-System, einschließlich einer M 510-Pumpe, eines WISP 710B-Autoinjektors, eines LC-Spektrophotometers M 481 und eines SHIMADZU-Integrators C-R3A, verwendet.

Säurehydrolyse und Aminosäureanalyse

Ca. 1 nMol des Proteins wird in ein Pyrexröhrchen gegeben. Dazu werden 200 $\mu$l 6M HCl konstant siedende HCl , welche 0,5 % 2-Mercaptoethanol enthält (I.T. Potts Jr. 1969, Anal. Biochem. 131, 1-15)- ,zugefügt. Die Röhrchen werden unter Vakuum versiegelt und 22 h bei 110°C inkubiert. Die Hydrolysate werden schnell getrocknet, erneut aufgelöst in 150 $\mu$l 0,2 M Natriumcitratpuffer, pH 2,2, und filtriert. Die Aminosäureanalysen werden mit einem BIOTRONIK LC 5000-Aminosäureanalysator durchgeführt, der mit einem Fluoreszenzdetektor und einem SHIMADZU C-R2AX-Integrator ausgestattet ist. Die Aminosäuren werden nach Umsetzung mit o-Phthaldialdehyd quantitativ bestimmt, wie dies im wesentlichen von Benson beschrieben ist (J. R. Benson, P.E. Hare 1975, Proc. Natl. Acad. SCI. USA 72, 619-622).

Standard-Leukozyten-Elastase-Assay

Materialien

Humane Leukozytenelastase wurde von Elastin Products Company, Inc., P.O. Box 147, Pacific, Miss. 63069/USA, bezogen.

Methoxysuccinyl-L-alanyl-L-alanyl-L-prolyl-L-valin-p-nitroanilid [K. Nakajima, J.C. Powers, M.J. Castillo, B.M. Ashe und M. Zimmermann, J. Biol. Chem. 254, 4027 (1979)] wurde von der Firma Bachem, Feinchemikalien AG, Hauptstrasse 144, CH-4416 Bubendorf/Schweiz, erworben.

Verfahren

Zu 550 $\mu$l eines Gemisches aus 0,2 M Tris-Puffer, pH 8,0, welcher 0,05 % Tween 80 und 0,05 M Kalziumchlorid enthielt, und der Lösung des Inhibitors, wurden 5 $\mu$l einer Lösung zugegeben, die erhalten wurde durch Auflösen von 1 mg des Enzyms in 100 ml 50 %igem Ehylenglykol. Das Gemische wurde 30 min bei Raumtemperatur inkubiert. Dann wurden unter Rühren 100 $\mu$l eines Gemisches aus 6,5 $\mu$l der Lösung von 59 mg Methoxysuccinyl-L-alanyl-L-alanyl-L-propyl-L-valin-p-nitroanilid in 1 ml Dimethylsulfoxid und 0,35 $\mu$l Testpuffer zugegeben, womit die Zunahme der optischen Dichte bei 405 registriert wurde. Die prozentuale Inhibierung wurde nach folgender Formel ermittelt:

$$\frac{\Delta\ E_{405}\ \text{des Ansatzes}}{\Delta\ E_{405}\ \text{der Enzymkontrolle}} \times 100.$$

Inhibierung von Trypsin

Die Hemmung von Trypsin wurde mit einen der Substrate Benzoyl-DL-arginin-p-nitroanilid (Merck 1670) oder Pyroglutamyl-glycyl-arginin-p-nitroanilid bestimmt, das aus im Handel erhältlichem Pyroglutamyl-glycin (SENN 6886) und Arginin-p-nitroanilid x HBr (SENN 9123) mit Hilfe der Dicyclohexyl-carbodiimid-Kondensaationsmethode synthetisiert wurde. Dieses Substrat wird auch unter der Bezeichnung S-2444 von KABI als ein Urokinasesubstrat vertrieben.

Das erstere hat den Vorteil, daß man zur Aprotininmenge in der Probe eine lineare Wirkung erhält, nachteilig ist jedoch eine geringe Empfindlichkeit. Letzteres besitzt eine hohe Empfindlichkeit; aufgrund der Dissoziation des Aprotinin-Trypsin-Komplexes bei derart niedrigen Konzentrationen ist jedoch die Dosis-Wirkungs-Kurve nicht linear.

Der Puffer zur Bestimmung von Trypsin und Trypsininhibitoren ist 0,2 M Tris, pH 8,0, welcher 0,01 M $CaCl_2$ und 0,05 % Tween 80® enthält.

Die Bestimmungen können in einem beliebigen Photometer durchgeführt werden, das die Ablesung der optischen Dichte (OD) bei 400 nm erlaubt. Zur voll-automatischen Bestimmung müssen die Photometer ausgestattet sein mit einem Mikroprozessor, oder sie müssen mit einem geeigneten Personalcomputer ausgerüstet sein.

Für sämtliche Assays wurden 1 cm Halbmikro-Einweg-Plastik-Küvetten verwendet. OD-Werte werden in Intervallen von 1 min über 8 Zyklen bei Raumtemperatur abgelesen. Die durchschnittliche OD-Zunahme pro Minute wird willkürlich als Aktivitätseinheit angenommen.

Für Inhibierungsbestimmungen wird Schweine-Trypsin-Lösung (Merck 8350) in 0,001 N HCl/50 %igem Glycerin mit der Inhibitorprobe vermischt, mit Puffer auf 500 $\mu$l aufgefüllt und 10 min bei Raumtemperatur inkubiert. Die Reaktion wird durch Zugabe der Substratlösung initiiert. Genauere Angaben gehen aus der folgenden Tabelle hervor. Die Inhibierungswirkung wird aus einer Kalibrierungskurve entnommen oder mit Hilfe von Computerprogrammen, die speziell für diesen Zweck entwickelt wurden, automatisch berechnet.

|  | Assay mit Pyrglu-Gly-Arg-pNA als Substrat | Assay mit Benzoyl-Arg-pNA als Substrat |
|---|---|---|
| Trypsin Substrat: | 15 $\mu$l (1 $\mu$g/ml) 50 $\mu$l (0,02 M in 10 % Ethanol) | 20 $\mu$l (100 $\mu$g/ml) 50 $\mu$l (0,012 M in 10 % DMSO) |

Derzeitig kennt man eine große Zahl von verschiedenen Mikroorganismen, die für die Transformation geeignet sind; nämlich einzellige Organismen, die in Kulturen oder Fermentationsbrühen gezüchtet werden können. Bevorzugte Organismen zur Transformation schließen Bakterien, Hefen und Pilze.ein.

Der für die hier beschriebenen Arbeiten verwendete spezielle Organismus war E. Coli RR1$\Delta$M15, der bei der American Type Culture Collection unter der ATCC Nr. 35102 hinterlegt wurde. Es können auch andere geeignete E. coli Stämme verwendet werden.

Die vorliegende Erfindung umfaßt pharmazeutische Zubereitungen, die außer nicht-toxischen, inerten, pharmazeutisch geeigneten Exzipienten eine oder mehrere Verbindungen gemäß der Erfindung umfassen oder aus einer oder mehreren aktiven Verbindungen gemäß der Erfindung bestehen, sowie Verfahren zur Herstellung dieser Zubereitungen.

Die vorliegende Erfindung umfaßt auch pharmazeutische Zubereitungen in Dosiseinheiten. Dies bedeutet, daß die Zubereitungen in Form individueller Teile vorliegen, z. B. als Tabletten, beschichtete Tabletten, Kapseln, Pillen, Suppositorien und Ampullen, deren Gehalt an Wirkstoff einer Fraktion oder einem Vielfachen einer individuellen Dosis entspricht. Die Dosiseinheiten können z. B. enthalten: eine, zwei, drei oder vier individuelle Dosen oder eine Hälfte, ein Drittel oder ein Viertel einer individuellen Dosis. Eine individuelle Dosis enthält vorzugsweise die Menge an Wirkstoff, die bei einer Verabreichung gegeben wird, und die gewöhnlich dem Ganzen, der Hälfte oder einem Drittel oder einem Viertel der Tagesdosis entspricht.

Unter nicht-toxischen, inerten, pharmazeutisch geeigneten Exzipienten sind zu verstehen: feste, halbfeste oder flüssige Verdünner, Füller und Formulierungshilfsmittel aller Art.

Tabletten, beschichtete Tabletten, Kapseln, Pillen, Granalien, Suppositorien, Lösungen, Suspensionen und Emulsionen, Pasten, Salben, Gele, Cremes, Lotionen, Pulver und Sprays können als bevorzugte pharmazeutische Zubereitungen genannt werden.

Tabletten, beschichtete Tabletten, Kapseln, Pillen und Granalien können den Wirkstoff oder die Wirkstoffe zusammen mit üblichen Exzipienten, wie (a) Füller und Streckmittel, z. B. Stärken, Lactose, Sucrose, Glucose, Mannit und Siliziumoxid, (b) Bindemittel, z. B. Carboxymethylcellulose, Alginate, Gelatine und Polyvinylpyrrolidon, (c) anfeuchtende Mittel, z. B. Glycerin, (d) disintegrierende Mittel, z. B. Agar-Agar, Kalziumcarbonat und Natriumcarbonat, (e) Lösungsverzögerer, z. B. Paraffin und (f) Absorptionsbeschleuniger, z. B. quaternäre Ammoniumverbindungen, (g) Benetzungsmittel, z. B. Cetylalkohol und Glycerinmonostearat, (h) Absorbentien, z. B. Kaolin und Bentonit und (i) Schmiermittel, z. B. Talk, Kalzium- und Magnesiumstearat und feste Polyethylenglykole, oder Gemische der unter (a) bis (i) aufgeführten Substanzen, enthalten.

Die Tabletten, beschichteten Tabletten, Kapseln, Pillen und Granalien können mit üblichen Überzügen und Umhüllungen versehen werden, die gegebenenfalls Opazifizierungsmittel enthalten; sie können eine Zusammensetzung aufweisen, so daß die Freisetzung des Wirkstoffes oder der Wirkstoffe nur, oder vorzugsweise, in einem bestimmten Teil des Intestinaltraktes, gegebenenfalls in verzögerter Form, erfolgt, wobei Beispiele für geeignete Zubereitungen zum Einbetten polymere Substanzen und Wachse bilden.

Die aktive Verbindung oder Verbindungen, gegebenenfalls zusammen mit einem oder mehreren der vorstehend genannten Exzipienten, kann bzw. können auch in mikroverkapselter Form vorliegen.

Suppositorien können außer dem Wirkstoff bzw. den Wirkstoffen enthalten: übliche wasserlösliche oder wasserunlösliche Exzipienten, z. B. Polyethylenglykole, Fette, z. B. Kakaofett und höhere Ester (z. B. $C_{14}$-Alkohol mit $C_{16}$-Fettsäure) oder Gemische dieser Substanzen.

Salben, Pasten, Cremes und Gele können zusätzlich zu dem Wirkstoff oder den Wirkstoffe übliche Exzipienten enthalten, z. B. tierische und pflanzliche Fette, Wachse, Paraffine, Stärke, Tragacanth, Cellulosederivate, Polyethylenglykole, Silikone, Bentonite, Siliziumdioxid, Talk und Zinkoxid, oder Gemische dieser Substanzen.

Pulver und Sprays können außer dem Wirkstoff oder den Wirkstoffen übliche Exzipienten enthalten, z. B.: Lactose, Talk, Siliziumdioxid, Aluminiumhydroxid, Kalziumsilikat und Polyamidpulver, oder Gemische dieser Substanzen. Sprays können außerdem die üblichen Treibmittel enthalten, z. B. Chlorfluorkohlenwasserstoffe.

Lösungen und Emulsionen können außer dem Wirkstoff oder den Wirkstoffen übliche Exzipienten umfassen, wie Lösungsmittel, solubilisierende Agentien und Emulgiermittel, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsamenöl, Erdnußöl, Maiskeimöl, Olivenöl, Rizinusöl und Sesamöl, Glycerin, Glycerinformal, Tetrahydrofurfurylalkohol, Polyethylenglykole und Fettsäureester von Sorbitan, oder Gemische dieser Substanzen.

Zur parenteralen Verabreichung können die Lösungen und Emulsionen auch in einer sterilen Form vorliegen, die mit Blut isoton ist.

Suspensionen können zusätzlich zum Wirkstoff oder den Wirkstoffen übliche Exzipienten enthalten, wie flüssige Verdünner, z. B. Wasser, Ethylalkohol oder Propylenglykol, Suspendierungsmittel, z. B. ethoxylierte Isostearylalkohole, Polyoxyethylensorbit und Sorbitanester, mikrokristalline Cellulose, Aluminium-metahydroxid, Bentonit, Agar-Agar und Tragacanth, oder Gemische dieser Substanzen.

Die genannten Formulierungsformen können auch enthalten: Farbstoffe, Konservierungsmittel und Additive, die den Geruch oder Geschmack verbessern, z. B. Pfefferminzöl und Eukalyptusöl, und Süßungsmittel, z. B. Saccharin.

Die therapeutisch wirksamen Verbindungen sollten in den vorstehend genannten pharmazeutischen Zubereitungen vorzugsweise in einer Konzentration von 0,1 bis 99,5 Gew.-%, vorzugsweise von ca. 0,5 bis 95 Gew.-%, bezogen auf das Gesamtgemisch, vorliegen.

Die vorstehend genannten pharmazeutischen Zubereitungen können zusätzlich zu den Verbindungen gemäß der Erfindung auch andere pharmazeutische Wirkstoffe enthalten.

Die vorstehend genannten pharmazeutischen Zubereitungen werden in üblicher Weise nach bekannten Verfahren hergestellt, z. B. durch Vermischen des Wirkstoffes oder der Wirkstoffe mit dem Exzipienten oder den Exzipienten.

Die Wirkstoffe oder die pharmazeutischen Zubereitungen können lokal, oral, parenteral, intraperitoneal und/oder rektal verabreicht werden, vorzugsweise erfolgt dies oral oder parenteral, wie z. B. intravenös oder intramuskular.

Im allgemeinen hat es sich sowohl in der Humanmedizin als auch in der Tiermedizin als vorteilhaft erwiesen, den Wirkstoff oder die Wirkstoffe gemäß der Erfindung in Gesamtmengen von ca. 0,5 bis ca. 500, vorzugsweise von 5 bis 100 mg/kg Körpergewicht alle 24 h, gegebenenfalls in Form von mehreren individuellen Verabreichungen, zu geben, um die besten Ergebnisse zu erzielen. Eine individuelle Verabreichung enthält den Wirkstoff oder die Wirkstoffe gemäß der Erfindung vorzugsweise in Mengen von ca. 1 bis ca. 250, insbesondere 3 bis 60 mg/kg Körpergewicht. Es kann jedoch erforderlich sein, von der genannten Dosis abzuweichen, insbesondere in Abhängigkeit von der Natur und dem Körpergewicht des zu behandelnden Individuums, der Natur und Schwere der Krankheit, der Art der Zubereitung und der Verabreichung der Medizin, und der Zeit oder dem Intervall, über den die Verabreichung stattfindet.

So kann es in einigen Fällen genügen, mit weniger als der vorstehend genannten Menge an Wirkstoff auszukommen, während in anderen Fällen die vorstehend genannte Menge an Wirkstoff überschritten werden muß. Die speziell erforderliche optimale Dosis und die Verabreichungsart der Wirkstoffe kann in einfacher Weise vom Fachmann auf der Basis seines Fachwissens entschieden werden.

E. coli, transformiert mit Expressionsplasmiden pRK 49.2.1 und pRK 48.1.1 wurden bei der Deutschen Sammlung von Mikroorganismen, Grisebachstraße 8, D-3400 Göttingen, unter den Hinterlegungsnummern DSM 3678 und DSM 3679 hinterlegt.

Die Arg-15-Homologen wie beispielsweise eins im Beispiel 7 beschrieben ist, sind sehr gut zur Behandlung von akuten Entzündungen, wie rheumatroider Arthitis, hereditäres angioneurotisches Ödem, Pneumonie, Pankreatitis und von Schockzuständen einsetzbar. Weiterhin haben die Arg-15-Homologen eine protektive Funktion bei Dialysen mit Hilfe von künstlichen Membranen und allgemein bei der extrakorporalen Zirkulation.

Beispiele

Beispiel 1

Synthese und Reinigung von DNA-Fragmenten, die für Glu-52- und Val-15-Glu-52-Aprotinin kodieren

Die Oligonukleotide, die das Gen bilden, wurden mit Hilfe von synthetischen Festphasen-Methoden hergestellt. Das Syntheseschema für die Oligomeren entsprach dem Beschriebenen. Es wurden protonenaktivierte, geschützte 2'-Desoxyribonukleotid-phosphoramidite verwendet. Sämtliche Folgeschritte wurden automatischmit einem Applied Biosystems Model 380 DNA-Synthese-Apparat unter Verwendung geschützter Nukleotid, Lösungsmittel, Chemikalien und Reagentien, die vom gleichen Hersteller bezogen wurden, durchgeführt. Der Festphasen-Träger, der auch von diesem Hersteller war, aus kontrolliertem Porenglas, enthielt das Ausgangs-3'-Nukleotid. Bestimmte Modifikationen des automatisierten Reaktionszyklus wurden in Übereinstimmung mit den "Manufacturers Operating Instructions" und den "Users Bulletins" durchgeführt. Nach Beendigung der Synthese wurden die Oligomeren entblockt und vom festen Träger innerhalb des DNA-Synthesators entsprechend den Anweisungen des Herstellers abgespalten.

Die endgültige Entfernung der blockierenden Gruppen erfolgte durch Erwärmen der wäßrigen Lösung, die das Oligomer enthielt, mit konzentriertem Ammoniumhydroxid für 4 bis 24 h bei 55°C in einer versiegelten Ampulle. Die erhaltene Lösung wurde eingedampft, der Rückstand in 0,01 M Triethylammonium-bicarbonatpuffer, pH 7,0 (TEAB-Puffer) aufgelöst. Diese Lösung wurde über Sephadex-G 50® Gelfiltrationsharz chromatographiert. Die Säule wurde mit dem gleichen TEAB-Puffer equilibriert und eluiert. Das mit dem Leervolumen eluierende Material wurde vereinigt und die Lösung eingedampft.

Ein Teil des Rückstandes (10 bis 40 % der Extiktionseinheiten bei 260 nm), aufgelöst in Probenpuffer (Zusammensetzung: 0,1 % Bromphenol-Blau, 0,1 % Xylol-Cyanol, 10 mM Dinatrium-EDTA, in Formamid), wurde im weiteren durch Elektrophorese in Polyacrylamidgelen gereinigt. Die Gelgröße betrug 18 x 32 cm mit einer Dicke von 1,5 mm. Die Breite der Vertiefung für jedes auf diese Weise gereinigte Oligomer betrug 2 bis 5 cm; mit Hilfe eines einzigen Gels wurden bis zu fünf Oligomere gereinigt. Die Acrylamidkonzentration im Gel variierte von 14 bis 20 %, je nach der Kettenlänge des gewünschten Produktes. Für längere Oligomere wird ein 14 %iges Acrylamidgel bevorzugt, während kürzere Oligomere auf einem Acrylamidgel bis zu 20 % gereinigt werden. Die Gele enthalten auch 7 M Harnstoff und Tris-Borat-EDTA-Puffer (0,1 M Tris, 0, 1 M Borat, 2 mM EDTA, pH 8,3). Der Laufpuffer bestand aus den gleichen Tris-Borat-EDTA-Gemisch. Die Elektrophorese wurde bei 20 bis 60 W, konstanter Stromstärke, 6 bis 18 h durchgeführt. Standardisierte Techniken können aus verschiedenen "User Information Bulletins", wie sie von Applied Biosystems erhältlich sind, entnommen werden.

Nach Beendigung der Elektrophorese wird das Gel auf eine Plastikhülle gelegt und die Oligomeren werden durch UV-Licht visualisiert. Diese Visualisierung wird erreicht, indem man das Gel auf eine Fluoreszenz-Dünnschichtchromatographie-Platte legt und es mit einer kurzwelligen UV-Lichtquelle betrachtet. Das gewünschte Produkt ist das am langsamsten wandernde, blaue Haupt-DNA-Fragment. Die gewünschte Bande wird aus dem Gel herausgeschnitten. Das DNA-Oligomer wird aus dem Gelscheibchen auf pulverförmige Diethylaminoethyl(DEAE)cellulose gelegt und unter Verwendung eines Epi-Gene D-Gel$^R$ -Elektrophoresegerätes eluiert. Aus der Cellulose wird das Oligomer durch Elution mit 1 M TEAB-Puffer gewonnen. Die Pufferlösung, die das Oligomer enthält, wird eingedampft, der Rückstand in 0,01 M TEAB-Puffer aufgelöst und dann entsalzt, indem er über eine Sephadex-G 50$^R$-Säule, wie vorstehend beschrieben, chromatographiert wird. Das im Leervolumen eluierende Material wird vereinigt und unter Erhalt des Endproduktes lyophilisiert.

Unter Anwendung der vorstehend beschriebenen Verfahren wurden ca. 0,5 bis 5,0 A260-Einheiten von jedem der gereinigten Oligomere erhalten.

Beispiel 2

Konstruktion eines synthetischen Aprotinin-Mastergens für Glu-52- und Val-15-Glu-52-Aprotinin

Die Konstruktion dieser spezifischen synthetischen Aprotiningene umfaßt das Zusammensetzen von 15 gereinigten Oligonukleotiden (s. Fig. 14). Die in Fig. 3 gezeigte DNA-Sequenz umfaßt Initiationscodon ATG, zwei Terminationscodons, TAG und TAA, die terminalen Restriktionsstellen Eco RI, Hind III und Bam HI und interne Restriktionsstellen. Die Wahl dieser Stellen erleichterte das Klonieren der Kodierungssequenz und deren Modifikation.

Die angewendete Konstruktion zur Herstellung dieses synthetischen Gens sah außer den Fragmenten die Verwendung von Polynukleotid-kinase, T4 DNA-Ligase und Restriktionsenzymen vor, wie dies im Detail in Materialien und Methoden beschrieben ist.

Fünfzehn gereinigte Oligonukleotidfragmente wurden in 50 mM TEABC (Triethylammonium-bicarbonat-Puffer, pH 7,5), Endkonzentration 10 pMol/$\mu$l, aufgelöst. Die Phosphoryiierung sämtlicher Fragmente erfolgte in vier separaten Abschnitten (Frag. 1,3; Frag. 2,4,6; Frag. 5,7,9,11,13; Frag. 8,10,12,14,16). Für präparative Zwecke wurden jeweils 80 pMol von jedem Fragment in einem Gemisch aus 1X PNK-Mix, 2 $\mu$M ATP, 0,5 uCl 32 P gamma ATP pro 10 pMol Fragment, 10 Einheiten PNK pro pMol Fragment aufgelöst, so daß die Gesamtvolumen für Frag. 1,3 300 $\mu$l, für Frag. 2,4,6 400 $\mu$l, für Frag. 5,7,9,11,12 und Frag. 8,10,12,14,16 700 $\mu$l betrugen. Die Reaktion für jeden Abschnitt wurde 30 min bei 37°C durchgeführt. Sämtliche Abschnitte wurden phenolisiert, mit Ethanol präzipitiert, gewaschen und getrocknet.

Für Hybridisierungszwecke wurden Frag. 1,3 und Frag. 2,4,6 (Block A) aufgelöst und in 1X Ligase-Mix, Gesamtvolumen 120 $\mu$l, 5 min bei 70°C inkubiert, und innerhalb von 5 h auf Raumtemperatur abgekühlt. Die anderen Fragmente (Block B) wurden nach dem gleichen Verfahren in 240 $\mu$l hybridisiert.

Für Ligierungszwecke wurde die Block A-Lösung mit 12 $\mu$l 10 mM ATP, 12 $\mu$l 100 mM DTE, 20 $\mu$l TA-DNA-Ligase und die Block B-Lösung mit dem Doppelten dieser Menge ergänzt. Die Reaktion wurde 7 h bei 14°C durchgeführt. Danach wurden 10 $\mu$l T4 DNA-Ligase zu Block A und 20 $\mu$l zu Block B zugegeben und wiederum 45 min bei 14°C inkubiert. Die Gemische wurden phenolisiert, mit Ethanol präzipitiert und getrocknet.

Der erhaltene Block A wurde in 90 $\mu$l 1X SP-100 und 10 $\mu$l Eco RI (10 $\mu$/$\mu$l), Block B in 90 $\mu$l 1X SP-50 und 10 $\mu$l Bam HI aufgelöst und 1 h bei 37°C inkubiert. Die Reaktionen wurden durch Phenolextraktion und Ethanolpräzipitation gestoppt, dann wurde eine 6 %ige Polyacrylamid-Gelelektrophorese durchgeführt und die DNA-Blöcke wurden nach dem gleichen Verfahren, wie oben beschrieben, gewonnen.

Gleiche Mengen an radioaktiv markiertem Block A und B wurden in Wasser aufgelöst, auf 1X Ligase-Mix eingestellt und wie vorstehend beschrieben hybridisiert, zur endgültigen Ligierung zu einem synthetischen Gen. Dazu wurden 3 $\mu$l 10 mM ATP, 3 $\mu$l 100 mM DTE, 3 $\mu$l T4 DNA-Ligase zu 22 $\mu$l Hybridisierungsgemisch zugegeben und 7 h bei 14°C inkubiert. Daraufhin wurde erneut 1 $\mu$l T4 DNA-Ligase zugegeben; diese Reaktion erfolgte 45 min lang bei 14°C. Das Ligierungsprodukt wurde durch Phenolextraktion und Ethanolpräzipitation gereinigt. Ein Standard-Restriktionsenzym-Abbau (Bam HI 1,5 $\mu$l, Eco RI 1,5 $\mu$l-Doppelabbau) wurde in SP-50 durchgeführt. Das Material wurde mit Phenol extrahiert; vor der Ethanolpräzipitierung wurde die wäßrige Lösung auf 3 mM $MgCl_2$ - 0,3 M Natriumacetat eingestellt. Dann wurde eine 6 %ige Polyacrylamid-Gelelektrophorese durchgeführt, und das Gen wurde nach dem gleichen Verfahren, wie oben beschrieben, gewonnen.

## Beispiel 3

### Konstruktion von rekombinanten Plasmiden pRK 63.1.1 und pRK 54.1.1

Das für die experimentelle Aprotinin-Klonierung ausgewählte Plasmid war pUC 8 (J. Vieira und J. Messing, 1982 Gene, 19, 259). Dieser Klonierungsvektor besteht aus einem von pBR 322-abgeleiteten Ampicillinasegen und dem "Origin" der DNA-Replikation, ligiert an einen Abschnitt des lac Z-Gens, der eine Anordnung nur einmal vorkommender Restriktionsenzym-Erkennungsstellen enthält. Wenn dieser Vektor in bestimmten lac⁻E. coli eingeführt wird, so bilden die Transformanden blaue Kolonien auf geeigneten Indikatorplatten. Die Klonierung von DNA-Fragmenten in eine der multiplen Restriktionsstellen, z. B. zwischen Eco RI und Bam HI, inaktiviert das lac-Gen und führt dadurch zur Bildung weißer Kolonien.

### Vektorherstellung

Zur Ligierung von synthetischem Aprotinin-Mastergen in pUC 8 wurde eine präparative Vektor-Präparation hergestellt. Gereinigte pUC 8 DNA (30 pMol) wurde zweimal mit Eco RI und Bam HI unter Standard-Restriktionsendonuklease-Abbaubedingungen behandelt, um ein kleineres inneres Eco RI - Bam HI-Fragment herauszuschneiden. Diese Präparation wurde mit Phosphatase aus Kälberdarm dephosphoryliert, wie dies vorstehend beschrieben ist, durch Agarose-Gelelektrophorese aufgetrennt und dann das große Eco RI - Bam HI-Fragment des Vektors gereinigt (Standardbedingungen). Dieses Verfahren erleichtert enorm die weitere Arbeit mit dem Vektor, da die Selbstligierung der Vektormoleküle an die Eco RI- und Bam HI-Enden ausgeschlossen ist und der Untergrund an Transformanden drastisch reduziert ist.

Ligierung

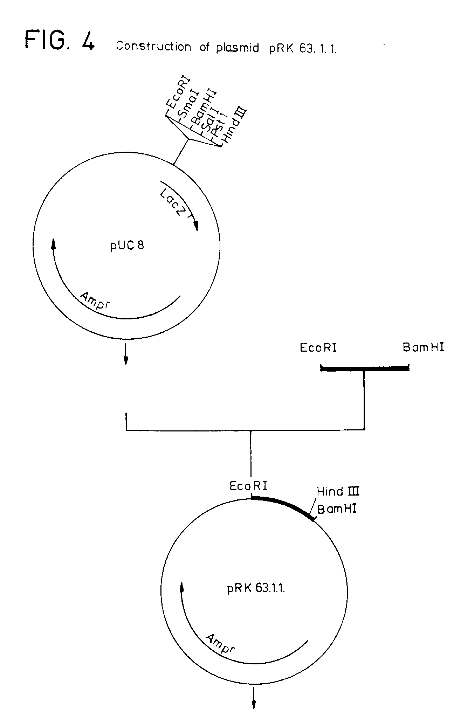

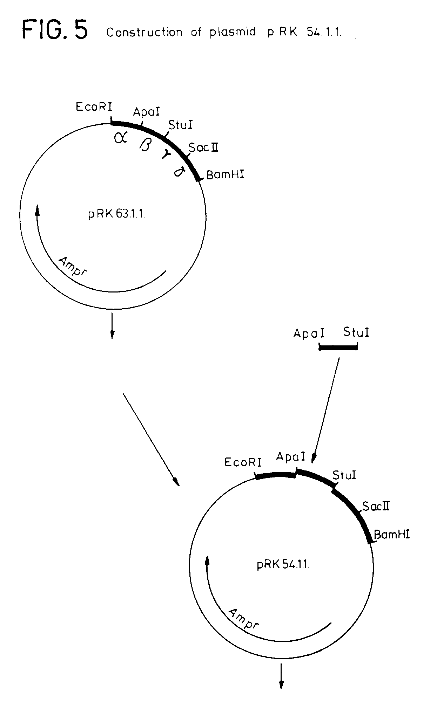

Die Konstruktion von pRK 63 (s. Fig. 4) erfolgte durch Ligieren der Gesamtmenge an gereinigtem synthetischen Aprotiningen mit 1 pMol Vektor (1,8 Einheiten T4-DNA-Ligase, 1X Ligase-Mix, Gesamtvolumen 45 µl, Inkubation 7 h bei 14°C, Zugabe 1 Einheit T4-DNA-Ligase und Reinkubation 45 min bei 14°C).

Transformation

Unter Anwendung des Transformationsverfahrens von D. Hanahan (Details siehe unter "Standard-Transformationsverfahren) wurde E. coli Stamm RRI delta M15 (A. Kalnins et al (1983), EMBO Journal 2, 593; ATCC 35102) als Empfänger verwendet. Nach Transformation wurden 15 "weiße" Transformanden,mit 50 % des Ligierungsmaterials auf Indikatorplatten, die 200 µg/ml Ampicillin enthielten, erhalten. Sämtliche 15 Transformanden wurden untersucht, wobei eine Modifikation der analytischen Schnell-Plasmidisolierungsmethode von Birnboim und Doly 1979 (siehe oben) angewendet wurde. Dementsprechend wurden Pellets der 15 Proben in 30 µl 1X SP-100, welche 1 µg RNase A enthielten, gelöst. Daraufhin folgte Restriktionsbehandlung mit Eco RI und Bam HI.

Nach Gelelektrophorese wurde festgestellt, daß vier der fünfzehn Transformanden eine Plasmid-DNA enthielten, die das Eco RI - Bam HI-Fragment von ca. 200 Basenpaaren aufwies. Sämtliche Transformanden, die dieses Eco RI - Bam HI-Fragment aufwiesen, wurden in größerer Menge gezüchtet und die Plasmide wurden jeweils isoliert und weiter analysiert. Zwei davon wurden nach dem Verfahren von Maxam und Gilbert sequenziert. Beide zeigten die korrekte Sequenz, was die ausgezeichnete Qualitat der chemischen Synthese und Konstruktion beweist.

Plasmid pRK 54.1.1 (Val-15-Glu-52-Aprotinin) wurde durch einen einfachen Austausch des beta-Blockes des snythetischen Gens, dies ist ein Apa I - Stu I-Fragment, durch einen beta-Block, der ein Codon für Val in Position 15 anstelle von Lys aufweist, konstruiert.

Ca. 100 pMol der synthetischen ss-DNA-Fragmente BEA 4A und BEA 4B (s. Fig. 2B) wurden in 20 µl Wasser aufgelöst, 5 min auf 95°C erwärmt und langsam auf Raumtemperatur abgekühlt (5 h). Das hybridisierte, nicht-phosphorylierte Fragment wurde mit 1,5 pMol gereinigter DNA aus pRK 63.1.1., dem das Apa I - Stu I-Fragment fehlt, ligiert. Die Standardligierung erfolgte in 30 µl Ligierungs-Mix. Die Transformation von E. coli RRIΔM15 erfolgte mit 50 % des Ligierungsgemisches.Von 1500 Transformanden wurden 24 durch analytische Plasmidisolierung und Restriktionsanalyse untersucht. Sämtliche waren positiv; zwei davon wurden durch das M 13 Didesoxynukleotid-Seuqenzierungssystem von BioLabs, Beveryl, MA USA, sequenziert. Der Transformand pRK 54.1.1 wurde für die weiteren Experimente verwendet.

Beispiel 4

Konstruktion der Expressionsplasmide pRK 48.1.1 und pRK 49.2.1

Zur Expression von Aprotinin als ein Fusionsprotein wurde ein Plasmid konstruhiert, in welchem das Aprotiningen am Carboxyende des $\beta$-Galactosidasegens lag,
wie dies durch ähnliche Experimente von U. Rüther und B. Müller-Hill 1983, EMBO Journal, 2, 1791-1794 und in DE-OS 33 09 501.9 gezeigt wurde.

Zur Klonierung des synthetischen Aprotiningens in dem Expressionsvektor pUR 278 wurden die Klonierungsstellen Bam HI und Hind III gewählt. Es war daher erforderlich, daß Aprotiningen durch Zufügen einer Bam HI-Stelle am 5'-Eco RI-Ende des Gens zu verwenden und die Hind III-Stelle an 3'-Ende zu verwenden (siehe auch Fig. 6).

50 pMol pRK 63.1.1 DNA wurden über Nacht bei 37°C mit Eco RI (15 pMol hit/µl) in 120 µl 1X SP-100 vollständig gespalten. Die überstehenden 5' Eco RI-Enden dieser DNA wurden durch eine enzymatische Reaktion mit DNA-Polymerase I (Klenow-Fragment), dATP und dTTP (Maniatis et al 1982) aufgefüllt. 600 pMol von getrocknetem alpha 32 P ATP (250 µCi) wurden aufgelöst und mit 160 µl DNA (50 pMol), 10 µl 1 mM dATP (10.000 pMol), 20 µl NT-Puffer, vermischt. Dann wurden 10 µl DNA-Polymerase I (Klenow, 50 µ) zugegeben und eine erste Inkubation bei Raumtemperatur durchgeführt. Nach 30 min wurden 10 µl 1 mM dTTP (10.000 pMol) zusammen mit 5 µl Polymerase (25 µ) zugegeben und eine zweite Inkubation bei Raumtemperatur durchgeführt. Das Material wurde mit Phenol/Sevag extrahiert, die radioaktiv-markierten Molekulargewichtsfraktionen wurden vereinigt, mit Ethanol ausgefällt, gewaschen, in 50 µl TE aufgelöst und bei 20°C gelagert.

20 µl dieses Materials mit glatten Enden wurden zur Ligierung mit Bam HI-Linker verwendet. Dazu wurden 400 pMol 5'-Bam HI-Linker, markiert mit gamma 32 P ATP (Standard-5'-Phosphorylierungsverfahren), mit 40 pMol DNA-Enden (Standard-Ligierungsbedingungen, 4,5 μ T4-DNA-Ligase, Gesamtvolumen 60 μl) ligiert. Um die Qualität der Linkerligierung zu kontrollieren, wurde eine analytische Gelelektrophorese durchgeführt. Eine vollständige Ligierung wurde nach Zugabe von 100 pMol Bam HI-Linker, 1,8 Einheiten T4-DNA-Ligase, Inkubation 1 h bei 20°C und Zugabe einer Einheit T4-DNA-Ligase und 18-stündiger Inkubation bei 14°C erzielt. Das Reaktionsgemisch wurde mit Phenol/Sevag extrahiert, mit Ethanol ausgefällt, gewaschen, getrocknet und in 40 μl TE aufgelöst.

Zur Herstellung des synthetischen Aprotiningens mit Bam HI- und Hind III-Enden wurde das mit Linker versehene lineare Plasmid (10 pMol) zunächst mit Hind III (10,5 pMol hit/μl, 5 h, 37°C) und dann mit Bam HI (40 pMol hit/μl, 20 h, 37°C Standardbedingungen) gespalten. Das Fragment wurde nach Auftrennung über 6%ige Polyacrylamid-Gelelektrophorese isoliert und sorgfältig gereinigt (s. Standardverfahren).

## Vektorherstellung

Der Ausgangs- Vektor pUR 278 (ca. 5 pMol) wurde zuerst mit Hind III (Standardbedingungen) gespalten, durch Extraktion mit Phenol/Sevag gereinigt, mit Ethanol ausgefällt, erneut gelöst und dann mit Bam HI (Standardbedingungen) verdaut. Dieses Material wurde auf ein 1 %iges Agarosegel gegeben, einer Elektrolyse unterzogen, isoliert und nach den Standardbedingungen gereinigt, um das 18 Basenpaar lange Bam HI - Hind III-Fragment, das bei der Ligierung mit dem synthetischen Aprotiningen in Kompetition treten würde, abzutrennen.

## Ligierung und Transformation

Zur Ligierung wurden 0,3 pMol Vektor, 1,5 pMol Fragment (ca.), 2 Einheiten T4-DNA-Ligase verwendet (Standardbedingungen, Gesamtvolumen 30 μl, Inkubation 4 h bei 14°C).

Die Transformation erfolgte mit E. coli Stamm RR1 delta M 15 als Wirt, wobei ein Drittel des Ligierungsgemisches verwendet wurde (Standardbedingungen). Insgesamt wurden 173 "blaue" Kolonien auf Indikatorplatten erhalten, die 200 μg Ampicillin/ml enthielten. Von diesen wurden 12 Transformanden im weiteren durch analytische Schnellplasmidisolierung (Standardbedingungen) analysiert. Von 173 Transformanden sollten, berechnet entsprechend dem Prozentsatz von Transformanden, die durch Religierung des Vektors erhalten wurden, 30 Untergrund-Transformanden darstellen. Dieses Ergebnis wurde durch Restriktionsanalyse der Plasmide der 12 Transformanden bestätigt. Acht davon waren positiv und wiesen ein Bam HI- Hind III-Restruktionsfragment von ca. 200 Basenpaaren auf. Positive rekombinante Plasmide wurden auch durch Sst II, die einmal vorkommende Restriktionsstelle,die innerhalb des Aprotiningens liegt, linearisiert. Eine Basensequenzanalyse gemäß Maxam und Gilbert, 1980, zeigte, daß das Plasmid pRK 48.1.1 das gewünschte Aprotinin-DNA-Fragment insertiert hat (s. Fig. 6). Plasmid pRK 48.1.1 wurde zur weiteren Analyse und Expressionsarbeit verwendet.

Die Konstruktion von Plasmid pRK 49.2.1 erfolgte nach genau dem gleichen Verfahren, unter Verwendung des Val-15-Glu-52-Gens aus pRK 54.1.1. Das positive rekombinante Plasmid 49.2.1 wies die korrekte DNA-Sequenz auf; dieses Konstrukt wurde zur weiteren Analyse und Expressionsarbeit verwendet.

## Nachweis der Expression von β-Galactosidase - Glu-52-Aprotinin und β-Galactosidase - Val-15-Glu-52-Aprotinin

Zum Erhalt einer Expression eines jeden dieser Konstrukte wurden E. coli Stämme mit pRK 48.1.1 (hinterlegt bei DSM, DSM Nr. 3679) und pRK 49.2.1 (hinterlegt bei DSM, DSM Nr. 3678) in 2 ml LB-Ampicillinmedium, ergänzt mit 4 μl 0,1 M IPTG, angeimpft. Ein Klon, der pUR 278 ohne Aprotiningen-Insert, enthielt, wurde auch in Kulturmedium angeimpft, um für die Assays eine negative Kontrolle zu erhalten. Nach 12- bis 16-stündigem Wachstum bei 37°C unter Rühren wurden Proben von 1 ml direkt zur Inokulation von 100 ml LB-Ampicillinmedium verwendet. Nach 12- bis 16-stündigem Kultivieren bei 37°C unter Rühren wurden die Zellen durch 10-minütige Zentrifugation bei 5.000 Upm in einem Beckman JA 10-Rotor gesammelt.

Der direkte Nachweis der Fusionsproteine erfolgte mittels SDS-Polyacrylamid-Gelelektrophorese nach Laemmli, U.K. (1970, Nature, 277, S. 680, siehe auch B.D. Hames und D. Rickwood 1981, Gel electrophoresis of proteins, IRL Press Limited, Oxford).

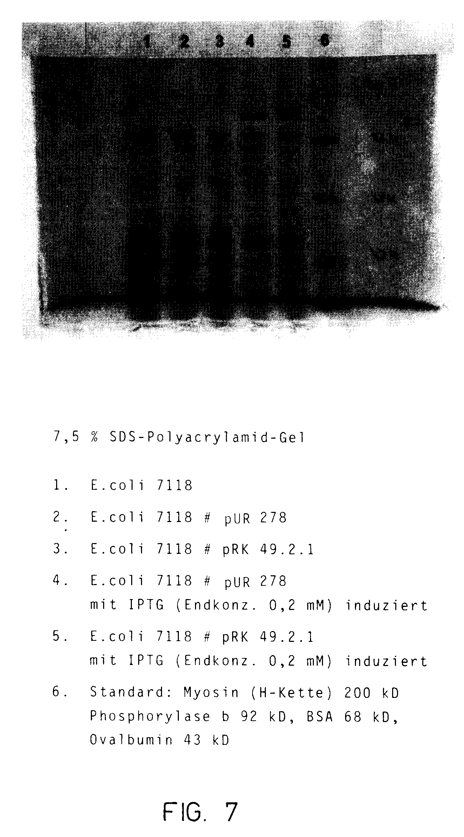

Pro Spur wurden ca. 1 x 10E9 Zellen zentrifugiert und in einer 1:5-Verdünnung von SDS-Probepuffer (0,3 M Tris.HCl, pH 8,8, 50 % Glycerin, 5 % SDS, 25 % Mercaptoethanol) erneut aufgelöst. Nach Elektrophorese wurden die Gele mit Coomassie-Bleau angefärbt. Fig. 7 zeigt ein typisches Bild von E. coli Proteinen mit dem induzierbaren β-Galactosidase-Glu-52-Aprotinin-Fusionsprotein.

Beispiel 5

Herstellung von Glu-52-Aprotinin

1. Isolierung und Bromcyan-Spaltung von $\beta$-Gal-fusionsprotein-Lys-15-Glu-52-Aprotinin

Lösungen:

Puffer zum Aufbrechen:
50 mM Tris
100 mM Natriumchlorid
10 mM Magnesiumchlorid
10 mM Mercaptoethanol
2 M Guanidinium-HCl-Lösung
2 M Guanidinium-HCl
50 mM Tris-HCl, pH 7,7
100 mM Natriumchlorid
10 mM Mercaptoethanol
6 M Guanidinium-HCl-Lösung
6 M Guanidinium-HCl
50 mM Tris-HCl
100 mM Natriumchlorid
10 mM Mercaptoethanol

Eine ausgewachsene 6 1-Übernachtkultur des E. coli Stammes RR1 $\Delta$ M15, mit dem Plasmid p RH UP. 1.1 15 min bei 8.000 Upm zentrifugiert wurde. Das Zellpellet mit einem Gewicht von ca. 15 g wurde in 30 ml Puffer zum Aufschließen resuspendiert und 6 min (unter Eiskühlung) beschallt. Das Zellysat wurde 20 min bei 20.000 Upm zentrifugiert. Der Überstand wurde verworfen. Das Pellet, ca. 10 g, wurde in 20 ml 2 M Guanidinium-hydrochlorid-Lösung resuspendiert und homogenisiert. Nach 20-minütiger Zentrifugation bei 20.000 Upm wurde der Überstand verworfen. Das Pellet, ca. 8 g, wurde unter einer $N_2$-Atmosphäre in 20 ml 6 M Guanidiniumhydrochlorid-Lösung, die 200 mg DTT enthielt, aufgelöst und 1 h bei 50°C reduziert. Die Lösung wurde 10 min bei 10.000 Upm zentrifugiert und 24 h gegen Wasser, das 10 mM Mercaptoethanol enthielt, dialysiert. Das ausgefällte Fusionsprotein wurde durch 20-minütige Zentrifugation bei 20.000 Upm gesammelt. Das feuchte Fusionsprotein wurde in 30 ml konzentrierter Ameisensäure aufgelöst und dann zu 70 % mit Wasser verdünnt. Das Fusionsprotein wurde durch Zugabe von 4 g Bromcyan gespalten und 18 h unter Stickstoffatmosphäre im Dunkeln inkubiert. Die Reaktion wurde durch Verdünnen mit 200 ml Wasser gestoppt. Das Wasser und die flüchtigen Nebenprodukte wurden durch Gefriertrocknen entfernt. Die Bromcyan-Spaltung wurde mittels SDS-Gelelektrophorese nach Laemmli überprüft. Die Ausbeute betrug ca. 1,5 g Bromcyan-Fragmente. In einer Reihe von Experimenten variierten die Ausbeuten von 0,8 bis 2,5 g.

Lösungen

Puffer A, pH 8,2
50 mMol Tris-HCl
1 mMol EDTA
eingestellt auf pH 8,2;
Puffer 8, pH 8,2
8 M Harnstoff
1 mMol Bis-(2-hydroxyethyl)-disulfid
1 mMol 2-Mercaptoethanol
in Puffer A;
Puffer C, pH 8,2
1 mMol Bis-(2-hydroxyethyl)-disulfid
1 mMol 2-Mercaptoethanol
in Puffer A;
Puffer D, pH 8,2
0,6 Mol Natriumchlorid in Puffer A.

2. Isolierung und Renaturierung von Glu-52-Aprotinin

Ca. 300 mg von gefriergetrocknetem Glu-52-Aprotinin-$\beta$-Galactosidase-Broncyan-Fragmenten wurden in 300 ml Puffer B, der 300 mg DTT enthielt, aufgelöst. Die Lösung wurde 1 h bei 50°C unter einer Stickstoffatmosphäre reduziert. Dann wurde die Lösung auf eine CM-Sephadex-Säule (25 x 100 mm) aufgegeben, die mit ca. 15 ml CM-Sepharose Fast Flow® gefüllt war. Die Säule war mit Puffer B equilibriert. Die Säule wurde mit Puffer B bis zum Erhalt einer stabilen Basislinie gewaschen. In einer ersten linearen Gradientenelution wurde die Säule mit 100 ml Puffer B und 100 ml Puffer C entwickelt.

Vor Aufgabe des zweiten linearen Elutionsgradienten wurde die Säule mit Puffer A bis zum Erhalt einer stabilen Basislinie gewaschen. Der zweite Gradient wurde gebildet aus 100 ml Puffer A und 100 ml Puffer D. Die Peakfraktionen wurden auf ihre Trypsin-inhibierende Aktivität sowie mittels ELISA und Western-Blot getestet (Fig. 10). In einer Reihe von Experimenten betrug die mittels der verschiedenen Tests ermittelte Ausbeute 0,4 bis 2 mg.

3. Reinigung von Glu-52-Aprotinin durch Umkehrphasen-HPLC

Die aktiven Fraktionen wurden durch Eindampfen konzentriert und dann gegen 0,1 M $NH_4 HCO_3$, pH = 7,5, 18 h dialysiert. Nach Lyophilisierung wurde der Inhibitor in 0,1 % TFA aufgelöst und durch Umkehrphasen-Chromatographie auf einer "wide-pore" RP - 18-Säule (BIORAD) unter Verwendung eines Gradienten aus 0,1 % TFA in Puffer A und 0,1 TFA 60 % in Puffer B fraktioniert. Der Inhibitor wurde durch N-terminale Sequenzierung und Aminosäureanalyse charakterisiert.

Beispiel 6

Expression von Val-15-Glu-52-Aprotinin und Charakterisierung des Produktes

Die Fermentation von E. coli- Zellen mit dem Plasmid pRK 49.2.1 und die Reinigung von Val-15-Glu-52-Aprotinin erfolgte nach dem gleichen Verfahren, wie es in Beispiel 5 beschrieben ist, mit dem Unterschied, daß die Aktivität mittels eines Elastase-inhibierenden Assays anstelle eines Trypsin-inhibierenden Tests bestimmt wurde. Val-15-Glu-52-Aprotinin eluiert früher von der CM-Se-phadexsäule als Glu-52-Aprotinin.

In einer Reihe von Experimenten wurde die Ausbeute durch verschiedene Tests zu 0,1 bis 1 mg ermittelt.

Zur Isolierung von Val-15-Glu-52-Aprotinin wurde die gleiche HPLC-Reinigung angewandt. Die inhibierende Wirkung wurde durch Messen der Leukozyten-Elastase-Inhibierung bestimmt. Der Inhibitor wurde durch N-terminale Sequenzierung und Aminosäureanalyse charakterisiert.

Beispiel 7

Konstruktion, Expression und Charakterisierung von Arg-15-Glu-52-Aprotinin

Das Arg-15-Glu-52-Aprotinin-Gen wurde durch Blockaustausch des ApaI-StuI DNA-Fragments im Val-15-Glu-52-Aprotinin-Gen (Plasmid pRK 54.1.1) hergestellt.

Das neu eingefügte Fragment, der Beta-Block des Aprotinin Master-Gens (s. auch Fig. 1), enthält das Arginin-Codon CGT an der Aminosäure Position 15. In dem nach Ligierung und Transformation neu entstandenen Vektor pNH 01.1.1 wurde die korrekte DNA-Sequenz des Gens bestätigt.

Zur Expression im Vektor pUR278 wurde an das 5'Ende des Gens eine "Bam HI site" angefügt, und das so veränderte Gen konnte in die Bam HI-HindIII-Schnittstellen des Expressionsvektors ligiert werden. Mit dieser DNA wurde E.coli RRI delta M15 transformiert und die positive Transformante mit dem neuen Vektor pRK 112.1.1 selektioniert.

Dieser E.coli-Stamm wurde vermehrt. Das $\beta$-Galactosidase Arg-15-Glu-52-Aprotinin-Fusionsprotein wurde isoliert. Daraus wurde nach der Bromcyan-Spaltung der Aprotininanteil gereinigt und näher charakterisiert.

Überraschenderweise zeigten kinetische Untersuchungen, daß gentechnologisch hergestelltes Arg-15-Glu-52-Aprotinin ein potenter Inhibitor von Plasma-Kallikrein ($K_i$ = $3.2 \times 10^{-10}$ M) und auch ein sehr guter Inhibitor von humanem kationischen und anionischen Trypsin mit $K_i$ -Werten, die unter $10^{-11}$ M liegen, ist. Die $K_i$ -Werte wurden mit synthetischen Substraten nach der Methode von M.W. Empie und M. Laskowski jr., Biochem. 21, 2274 (1982) bestimmt.

**Patentansprüche**
**Patentansprüche für folgende Vertragsstaaten : AT, BE, CH, DE, FR, GB, IT, LI, NL, SE**

1. Aprotinin, welches in Position 52 durch Glu, Leu, Val, Thr oder Ser und in Position 15 durch eine beliebige natürlich vorkommende Aminosäure substituiert ist.

2. Aprotinin gemäß Anspruch 1, welches in Position 15 durch Arg, Val, Ile, Leu, Phe, Gly, Ser, Trp, Tyr und Ala substituiert ist.

3. Aprotinin-Analoge aus der Gruppe Glu-52-Aprotinin, Val-15-Glu-52-Aprotinin, Arg-15-Glu-52-Aprotinin, Ile-15-Glu-52-Aprotinin und Leu-15-Glu-52-Aprotinin.

4. DNA, gekennzeichnet durch die folgende Sequenz:

```
      E                                                    A
      C                                                    P
      O                                                    A
      R                                                    I
      I
      AATTCATGCGTCCGGACTTCTGCCTCGAGCCGCCGTACACTGGGCCCTGCAAAGCTCGTA
   1  ---------+---------+---------+---------+---------+---------+ 60
      TTAAGTACGCAGGCCTTGAAGACGGAGCTCGGCGGACATGTGACCGGACGTTTCGAGCAT

  c :
                                        S
                                        T
                                        U
                                        1
      TCATCCGTTACTTCTACAATGCAAAGGCAGGCCTGTGTCAGACCTTCGTATACGGCGGTT
  61  ---------+---------+---------+---------+---------+---------+120
      AGTAGGCAATGAAGATGTTACGTTTCCGTCCGGACACAGTCTGGAAGCATATGCCGCCAA

  c :
                                        S
                                        A
                                        C
                                        2
      GCCGTGCTAAGCGTAACAACTTCAAATCCGCGGAAGACTGCGAACGTACTTGCGGTGGTC
 121  ---------+---------+---------+---------+---------+---------+180
      CGGCACGATTCGCATTGTTGAAGTTTAGGCGCCTTCTGACGCTTGCATGAACGCCACCAC

         H              B
         I              A
         N              M
         D              H
         3              I
      CTTAGTAAAGCTTG
 151  ---------+---- 194.
      GAATCATTTCGAAC
```

5. DNA, codierend für ein Aprotinin gemäß den Ansprüchen 1 - 3.

6. Expressionsplasmid, enthaltend die DNA nach Anspruch 5.

7. Wirtszellen, enthaltend das Plasmid nach Anspruch 6.

**8.** Pharmazeutische Zubereitung, dadurch gekennzeichnet, daß sie Aprotinin gemäß einem oder mehreren der Ansprüche 1 - 3 enthält.

**9.** Verwendung des Aprotinins gemäß den Ansprüchen 1 - 3 zur Herstellung von Arzneimitteln.

**Patentanspruch für folgenden Vertragsstaat : ES**

**1.** Verfahren zur Gewinnung von Aprotinin, welches in Position 52 durch Glu, Leu, Val, Thr oder Ser und in Position 15 durch eine beliebige natürlich vorkommende Aminosäure substituiert ist, gekennzeichnet durch

(a) Synthetisieren von DNA-Fragmenten, die für das Aprotinin kodieren,
(b) Ligieren der DNA-Fragmente unter Erhalt eines Master-Gens,
(c) Klonieren der Aprotinin-Master-Gene,
(d) Substituieren von DNA-Fragmenten in den Master-Genen,
(e) Ligieren der erhaltenen Master-Gene in einen geeigneten Expressionsvektor,
(f) Transformieren einer Wirtszelle mit dem genannten Expressionsvektor,
(g) Züchten von Wirtszellen,
(h) Isolieren des Fusionsproteins aus der Zellkultur,
(i) Spalten des Fusionsproteins und
(j) Isolieren von Aprotinin und Aprotinin-Homologen.

**Claims**
**Claims for the following Contracting States : AT, BE, CH, DE, FR, GB, IT, LI, NL, SE**

**1.** Aprotinin, which is substituted in position 52 by Glu, Leu, Val, Thr or Ser and in position 15 by any naturally occurring amino acid.

**2.** Aprotinin according to Claim 1, which is substituted in position 15 by Arg, Val, Ile, Leu, Phe, Gly, Ser, Trp, Tyr and Ala.

**3.** Aprotinin analogues from the group Glu-52-aprotinin, Val-15-Glu-52-aprotinin, Arg-15-Glu-52-aprotinin, Ile-15-Glu-52-aprotinin and Leu-15-Glu-52-aprotinin.

**4.** DNA, characterized by the following sequence:

```
        E                                              A
        C                                              P
        O                                              A
        R                                              I
        I
        AATTCATGCGTCCGGACTTCTGCCTCGAGCCGCCGTACACTGGGCCCTGCAAAGCTCGTA
     1  ---------+---------+---------+---------+---------+---------+ 60
        TTAAGTACGCAGGCCTTGAAGACGGAGCTCGGCGGACATGTGACCGGACGTTTCGAGCAT
```

22

```
        C:
                                            S
                                            T
                                            U
                                            1

   TCATCCGTTACTTCTACAATGCAAAGGCAGGCCTGTGTCAGACCTTCGTATACGGCGGTT
61 ------------------------------------------------------------·120
   AGTAGGCAATGAAGATGTTACGTTTCCGTCCGGACACAGTCTGGAAGCATATGCCGCCAA


        C:
                                            S
                                            A
                                            C
                                            2

   GCCGTGCTAAGCGTAACAACTTCAAATCCGCGGAAGACTGCTAACGTACTTGCGGTGGTC
121 -----------------------------------------------------------·130
   CGGCACGATTCGCATTGTTGAAGTTTAGGCGCCTTCTGACGCTTGCATGAACGCCACCAC


        H               B
        I               A
        N               M
        D               H
        3               I

   CTTAGTAAAGCTTG
151 ------------- 194.
   GAATCATTTCGAAC
```

5.  DNA, encoding an aprotinin according to Claims 1-3.

6.  Expression plasmid, containing the DNA according to Claim 5.

7.  Host cells, containing the plasmid according to Claim 6.

8.  Pharmaceutical preparation, characterized in that it contains aprotinin according to one or more of Claims 1-3.

9.  Use of the aprotinin according to Claims 1-3 for preparing medicaments.

**Claim for the following Contracting State : ES**

1.  Process for obtaining aprotinin which is substituted in position 52 by Glu, Leu, Val, Thr or Ser and in position 15 by any naturally occurring amino acid, characterized by
    (a) synthesizing DNA fragments which encode aprotinin,
    (b) ligating the DNA fragments, thereby obtaining a master gene,
    (c) cloning the aprotinin master genes,
    (d) substituting DNA fragments in the master genes,
    (e) ligating the master genes obtained into a suitable expression vector,
    (f) transforming a host cell with the said expression vector,
    (g) culturing host cells,
    (h) isolating the fusion protein from the cell culture,
    (i) cleaving the fusion protein, and
    (j) isolating aprotinin and aprotinin homologues.

23

**Revendications**
**Revendications pour les Etats contractants suivants : AT, BE, CH, DE, FR, GB, IT, LI, NL, SE**

1. Aprotinine, qui est substituée en position 52 par Glu, Leu, Val, Thr ou Ser et en position 15 par un amino-acide naturel quelconque.

2. Aprotinine suivant la revendication 1, qui est substituée en position 15 par Arg, Val, Ile, Leu, Phe, Gly, Ser, Trp, Tyr et Ala.

3. Analogues d'aprotinine du groupe Glu-52-aprotinine, Val-15-Glu-52-aprotinine, Arg-15-Glu-52-aprotinine, Ile-15-Glu-52-aprotinine et Leu-15-Glu-52-aprotinine.

4. ADN caractérisé par la séquence suivante :

```
      E                                              A
      C                                              P
      O                                              A
      R                                              I
      I
      AATTCATGCGTCCGGACTTCTGCCTCGAGCCGCCGTACACTGGGCCCTGCAAAGCTCGTA
1     ------------+---------+---------+---------+---------+--------+ 60
      TTAAGTACGCAGGCCTTGAAGACGGAGCTCGGCGGACATGTGACCGGACGTTTCGAGCAT

  c:
                                        S
                                        T
                                        U
                                        1

      TCATCCGTTACTTCTACAATGCAAAGGCAGGCCTGTGTCAGACCTTCGTATACGGCGGTT
61    --------+---------+---------+---------+---------+--------+120
      AGTAGGCAATGAAGATGTTACGTTTCCGTCCGGACACAGTCTGGAAGCATATGCCGCCAA

  c:
                                        S
                                        A
                                        C
                                        2

      GCCGTGCTAAGCGTAACAACTTCAAATCCGCGGAAGACTGCGAACGTACTTGCGGTGGTC
121   --------+---------+---------+---------+---------+--------+180
      CGGCACGATTCGCATTGTTGAAGTTTAGGCGCCTTCTGACGCTTGCATGAACGCCACCAC

          H        B
          I        A
          N        M
          D        H
          3        I

      CTTAGTAAAGCTTG
131   -----------+--- 194.
      GAATCATTTCGAAC
```

5. ADN codant pour une aprotinine suivant les revendications 1 à 3.

6. Plasmide d'expression, contenant l'ADN suivant la revendication 5.

7. Cellules-hôtes, contenant le plasmide suivant la revendication 6.

EP 0 238 993 B1

8. Préparation pharmaceutique, caractérisée en ce qu'elle contient l'aprotinine suivant une ou plusieurs des revendications 1 à 3.

9. Utilisation de l'aprotinine suivant les revendications 1 à 3 pour la préparation de médicaments.

**Revendication pour l'Etat contractant suivant : ES**

1. Procédé d'obtention d'aprotinine qui est substituée en position 52 par Glu, Leu, Val, Thr ou Ser et en position 15 par un amino-acide naturel quelconque, caractérisé par
   (a) la synthèse de fragments d'ADN qui codent pour l'aprotinine,
   (b) la ligature des fragments d'ADN avec obtention d'un gène-maître,
   (c) le clonage des gènes-maîtres de l'aprotinine,
   (d) la substitution de fragments d'ADN dans les gènes-maîtres,
   (e) la ligature des gènes-maîtres obtenus dans un vecteur d'expression approprié,
   (f) la transformation d'une cellule-hôte avec le vecteur d'expression mentionné,
   (g) la culture de cellules-hôtes,
   (h) l'isolement de la culture de cellules de la protéine fusionnée,
   (i) la scission de la protéine fusionnée, et
   (j) l'isolement de l'aprotinine et d'homologues de l'aprotinine.

25

Design of a synthetic Aprotinin Master Gene

FIG. 1

DNA-Sequence of Oligonucleotides used for
construction of synthetic genes consisting
of four blocks (alpha, beta, gamma, delta)

```
Fra    1    AATTCATGCGTCCGGACTTCTGCCTCGAGC
Fra    2    CAGAAGTCCGGACGCATG
Fra    3    CGCCGTACACTGGGCCCTGCAAAGCT
Fra    4    CCCAGTGTACGGCGGCTCGAGG
Fra    5    CGTATCATCCGTTACTTC
Fra    6    ATGATACGAGCTTTGCAGGG
Fra    7    TACAATGCAAAGGCAGGCCTGTGTCAGACC
Fra    8    CCTGCCTTTGCATTGTAGAAGTAACGG
Fra    9    TTCGTATACGGCGGTTGCCGTGCTAAGCGT
Fra   10    AACCGCCGTATACGAAGGTCTGACACAGG
Fra   11    AACAACTTCAAATCCGCGGAAGACTGCGAA
Fra   12    ATTTGAAGTTGTTACGCTTAGCACGGC
Fra   13    CGTACTTGCGGTGGTGCTTAGTAAAGCTTG
Fra   14    CACCGCAAGTACGTTCGCAGTCTTCCGCGG
Fra   16    GATCCAAGCTTTACTAAGCAC
```

# FIG. 2a

Sequence of the Beta-EA2-Block
(Leu-15 modification, CTG)
sites: Apal Stul

```
     CTGCCTGGCTCGTATCATCCGTTACTTCTACAATGCAAAGGCAGG
1    ---------+---------+---------+---------+-----   45
     GACGGACCGAGCATAGTAGGCAATGAAGATGTTACGTTTCCGTCC

     CysLeuAlaArgIleIleArgTyrPheTyrAsnAlaLysAla???
```

Sequence of the Beta-EA4-Block
(Val-15 modification, GTT)
sites: Apal-Stul

```
     CTGCGTTGCTCGTATCATCCGTTACTTCTACAATGCAAAGGCAGG
1    ---------+---------+---------+---------+-----   45
     GACGCAACGAGCATAGTAGGCAATGAAGATGTTACGTTTCCGTCC

     CysValAlaArgIleIleArgTyrPheTyrAsnAlaLysAla???
```

Sequence of the Beta-EA5-Block
(Ile-15 modification, ATC)
sites: Apal-Stul

```
     CTGCATCGCTCGTATCATCCGTTACTTCTACAATGCAAAGGCAGG
1    ---------+---------+---------+---------+-----   45
     GACGTAGCGAGCATAGTAGGCAATGAAGATGTTACGTTTCCGTCC

     CysIleAlaArgIleIleArgTyrPheTyrAsnAlaLysAla???
```

# FIG. 2b

28

DNA-Sequence of the synthetic Glu-52-Aprotinin Gene
(EcoR1-BamH1, partial-Sequence of pRK 63.1.1)

```
E                                              A
C                                              P
O                                              A
R                                              I
I
AATTCATGCGTCCGGACTTCTGCCTCGAGCCGCCGTACACTGGGCCCTGCAAAGCTCGTA
---------+---------+---------+---------+---------+---------+   60
TTAAGTACGCAGGCCTGAAGACGGAGCTCGGCGGCATGTGACCCGGGACGTTTCGAGCAT
```

c:        ArgProAspPheCysLeuGluProProTyrThrGlyProCysLysAlaArgIle

```
                       S
                       T
                       U
                       1
TCATCCGTTACTTCTACAATGCAAAGGCAGGCCTGTGTCAGACCTTCGTATACGGCGGTT
---------+---------+---------+---------+---------+---------+  120
AGTAGGCAATGAAGATGTTACGTTTCCGTCCGGACACAGTCTGGAAGCATATGCCGCCAA
```

c:        IleArgTyrPheTyrAsnAlaLysAlaGlyLeuCysGlnThrPheValTyrGlyGlyCys

```
                       S
                       A
                       C
                       2
GCCGTGCTAAGCGTAACAACTTCAAATCCGCGGAAGACTGCGAACGTACTTGCGGTGGTC
---------+---------+---------+---------+---------+---------+  180
CGGCACGATTCGCATTGTTGAAGTTTAGGCGCCTTCTGACGCTTGCATGAACGCCACCAG
```

          ArgAlaLysArgAsnAsnPheLysSerAlaGluAspCysGluArgThrCysGlyGlyAla

```
          H
          I        B
          N        A
          D        M
          3        HI
CTTAGTAAAGCTTG
---------+----   194
GAATCATTTCGAAC
```

FIG. 3

FIG. 4   Construction of plasmid pRK 63. 1. 1.

FIG. 5   Construction of plasmid p RK 54.1.1.

FIG. 6  Construction of  p RK 48.1.1.

7,5 % SDS-Polyacrylamid-Gel

1. E.coli 7118

2. E.coli 7118 # pUR 278

3. E.coli 7118 # pRK 49.2.1

4. E.coli 7118 # pUR 278
   mit IPTG (Endkonz. 0,2 mM) induziert

5. E.coli 7118 # pRK 49.2.1
   mit IPTG (Endkonz. 0,2 mM) induziert

6. Standard: Myosin (H-Kette) 200 kD
   Phosphorylase b 92 kD, BSA 68 kD,
   Ovalbumin 43 kD

FIG. 7

ISOLATION OF ß-GALACTOSIDASE FUSION PROTEIN

FROM HS 2074 CHECKED BY AN 8 % SDS-GEL UNDER

REDUCING CONDITIONS

200 kDal

97 kDal
66 kDal

44 kDal

26 kDal

MARKER

ß-GALACTOSIDASE

CELL SUSPENSION

CELL LYSATE

SUPERNATANT

FUSION PROTEIN AFTER
WASHING A 2 M GUANIDINIUM-
HCl SOLUTION

ß-GALACTOSIDASE

MARKER

FIG. 8

FIG. 9

Aprotinin

40 42 43 44 45 46 47 48    fraction

Westernblot of the fractionated cyanogen bromide
peptides of Lys15 Glu52-aprotinin-ß-galactosidase
fusionprotein

FIG. 10

FIG. 11

8 % SDS-Polyacrylamid gel according to Laemmli of
Val15 Glu52 aprotinin-ß-gal-fusionprotein

1. Standard Myosin (200kD), ß-Galactosidase (117 kD),
   Phosphorylase (92 kD), BSA (66 kD)-Ovalbumin (44 kD)

2. Cell-lysate (pRK 49.2.1)

3. Supernatant

4. Pellet

5. Val15 Glu52-aprotinin-ß-galactosidase-fusionprotein
   after reductive carboxymethylation

6. Standard Phosphorylase (92 kD), BSA (66 kD),
   Ovalbumin (44 kD), Lactoglobulin (18kD)

# FIG. 12

FIG. 13